(19) Europäisches Patentamt / European Patent Office / Office européen des brevets

(11) **EP 4 413 928 A1**

(12) **EUROPEAN PATENT APPLICATION**

(43) Date of publication:
**14.08.2024 Bulletin 2024/33**

(21) Application number: **24151592.3**

(22) Date of filing: **12.01.2024**

(51) International Patent Classification (IPC):
**A61B 6/00** (2024.01)      **G06T 11/00** (2006.01)
**A61B 6/03** (2006.01)      **G06T 5/50** (2006.01)
**G06T 5/60** (2024.01)

(52) Cooperative Patent Classification (CPC):
**G06T 11/006; A61B 6/032; A61B 6/482; A61B 6/5205; G06T 11/005**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **03.02.2023 US 202318164372**

(71) Applicant: **GE Precision Healthcare LLC**
**Waukesha, WI 53188 (US)**

(72) Inventors:
• **DAS, Bipul**
**560066 Bengaluru (IN)**

• **AGRAWAL, Utkarsh**
**560066 Bengaluru (IN)**
• **SUDHAKARA MURTHY, Prasad**
**560066 Bengaluru (IN)**
• **SHIGEMASA, Risa**
**Hino, 191-8503 (JP)**
• **OGATA, Kentaro**
**Hino, 191-8503 (JP)**
• **IMAI, Yasuhiro**
**Hino, 191-8503 (JP)**

(74) Representative: **Kilburn & Strode LLP**
**Lacon London**
**84 Theobalds Road**
**Holborn**
**London WC1X 8NL (GB)**

(54) **SYSTEMS AND METHODS FOR INTERPOLATION OF DUAL-ENERGY CT DATA**

(57) Methods and systems are provided for interpolating missing views in dual-energy computed tomography data. In one example, a method includes obtaining (302, 304) a first sinogram missing a plurality of views and a second sinogram missing a different plurality of views, the first sinogram acquired with a first X-ray source energy during a scan and the second sinogram acquired with a second, different X-ray source energy during the scan; initializing (306) each of the first sinogram and the second sinogram to form a first initialized sinogram and a second initialized sinogram; entering (312) the first initialized sinogram and the second initialized sinogram into the same or different interpolation models trained to output a first filled sinogram based on the first initialized sinogram and output a second filled sinogram based on the second initialized sinogram; and reconstructing (318) one or more images from the first filled sinogram and the second filled sinogram.

FIG. 1

EP 4 413 928 A1

**Description**

FIELD

Embodiments of the subject matter disclosed herein relate to medical imaging, and more particularly to interpolation of missing views in dual energy computed tomography data.

BACKGROUND

Certain types of computed tomography (CT) imaging systems may operate with more than one X-ray source energy, such as dual-energy CT imaging systems. In dual-energy CT imaging systems, the X-ray source energy may be switched multiple times over the course of a scan between high energy and low energy, for example. As a result, a portion of the total views obtained during the scan may be obtained with low energy while the remaining views may be obtained with high energy, leading to each energy level having less than complete view data. The missing views may be interpolated, but the interpolation may lead to image artifacts and/or image quality degradation.

BRIEF DESCRIPTION

In one example, a method includes obtaining a first sinogram and a second sinogram of an imaging subject, wherein the first sinogram is missing a plurality of views and the second sinogram is missing a different plurality of views, the first sinogram acquired with a first X-ray source energy during a scan and the second sinogram acquired with a second, different X-ray source energy during the scan; initializing the first sinogram with information from the second sinogram to form a first initialized sinogram; initializing the second sinogram with information from the first sinogram to form a second initialized sinogram; entering the first initialized sinogram into an interpolation model trained to output a first filled sinogram based on the first initialized sinogram, and entering the second initialized sinogram into the interpolation model or another interpolation model trained to output a second filled sinogram based on the second initialized sinogram; and reconstructing one or more images from the output first filled sinogram and the output second filled sinogram.

It should be understood that the brief description above is provided to introduce in simplified form a selection of concepts that are further described in the detailed description. It is not meant to identify key or essential features of the claimed subject matter, the scope of which is defined uniquely by the claims that follow the detailed description. Furthermore, the claimed subject matter is not limited to implementations that solve any disadvantages noted above or in any part of this disclosure.

BRIEF DESCRIPTION OF THE DRAWINGS

The present invention will be better understood from reading the following description of non-limiting embodiments, with reference to the attached drawings, wherein below:

FIG. 1 shows a pictorial view of an imaging system, according to an example.
FIG. 2 shows a block schematic diagram of an imaging system, according to an example.
FIG. 3 is a flow chart illustrating an example method for interpolating missing views in dual energy sinograms.
FIG. 4 schematically shows a first example network architecture for generating a single interpolated sinogram from dual energy sinograms with missing views.
FIG. 5 schematically shows a second example network architecture for generating two interpolated sinograms from dual energy sinograms with missing views.
FIG. 6 is a flow chart illustrating an example method for mixing high energy and low energy sinograms at the feature level.
FIG. 7 schematically shows an example architecture for feature mixing of two sinograms of different energy.
FIG. 8 is a flow chart illustrating an example method for interpolating views of a sinogram using a single-channel multi-scale interpolation model.
FIG. 9 schematically shows an example process for multi-scale interpolation.
FIG. 10 schematically shows a first example single-channel network architecture for performing multi-scale view interpolation.
FIG. 11 schematically shows a second example single-channel network architecture for performing multi-scale view interpolation.
FIG. 12 is a flow chart illustrating an example method for interpolating views of two sinograms using a dual-channel multi-scale interpolation model.
FIG. 13 schematically shows an example dual-channel network architecture for performing multi-scale view interpolation.
FIGS. 14 and 15 show example CT images that may be produced using the systems and methods described herein.

DETAILED DESCRIPTION

The following description relates to various embodiments for systems and methods for sparse view image reconstruction, and in particular interpolation of missing views of projection data obtained with dual-energy (DE) computed tomography (CT) imaging systems. DECT imaging systems may be configured to obtain projection data at two different X-ray source energies during a single scan of an imaging subject by switching the X-ray source energy multiple times during the scan. The energy switching results in the high-energy projection data (e.g., the views obtained with the X-ray source at a higher energy) missing views that were obtained at lower energy, and vice versa (such that the low-energy projection data also misses views that were obtained at the higher energy). As an example, if a complete scan includes the acquisition of 1000 views, 500 views may be acquired at the higher energy while the remaining 500 views may be acquired at the lower energy. During image reconstruction, each set of projection data/sinogram (e.g., the high-energy projection data and the low-energy projection data) may be used to generate basis material images, for example. To compensate for the missing views, classical or geometric interpolation may be carried out on each set of projection data and/or on the reconstructed images to fill in the missing views for each energy level. However, this approach often results in image artifacts and/or image quality degradation, particularly for slower-switching imaging systems where two or more consecutive views may be obtained at one energy level before switching to the other energy level (resulting in more than one consecutive missing view to be interpolated).

Thus, embodiments are disclosed herein to interpolate the missing views in each sinogram using a multi-scale hierarchical multi-tasking deep learning framework (referred to herein as a multi-scale interpolation model). The proposed model (or models depending on the network architecture) takes in low- and high-energy sinograms with missing views independently or in mixed mode as input and generates corresponding low and high energy sinograms, such that missing views are interpolated (e.g., filled in with data based on data from neighboring views). The multi-scale interpolation model may utilize a pyramid structure for multi-scale gradient optimization to ensure high precision at each scale of sinogram interpolation from a coarsest scale to a finest scale of complete sinogram at the highest resolution. To accomplish this, the multi-scale interpolation model is trained with loss functions calculated at each resolution level (rather than a single loss function calculated on the final output/highest resolution sinogram). In doing so, accurate sinogram completion may be achieved without having to further process the completed sinograms.

In some examples, the initial sinograms that are input to the multi-scale interpolation model(s) may be processed to include data from both the high energy sinogram and the low energy sinogram, such that information from the low-energy sinogram may be used to inform the interpolation of the missing views of the high-energy sinogram and information from the high-energy sinogram may be used to inform the interpolation of the missing views of the low-energy sinogram.

For example, for a set of sinograms acquired at different energies, such that more than one view is missing at regular intervals and alternate energies having complementary missing views, the multi-scale interpolation model(s) may be applied to interpolate the missing views for each sinogram. The model(s) may intelligently use complementary information from the alternate energy wherever applicable and learns to complete one or both sinograms in a supervised manner. Driven by X-ray attenuation physics, based on the material or tissues over which the rays are passing, the data acquired at two different energy levels may either have similar values or can have large differences. Some regions have similar values (like water, soft tissue, air etc.), while other regions like bone, iodine contrast, metal, etc., are non-similar between low energy (70/80 etc. kVp) and high energy (120-140 kVp). At lower energy, attenuation is predominantly determined by photoelectric effect and at higher energy attenuation is determined by the Compton effect. Thus, the relationship variation between the two energy levels is non-linear in nature. The model(s) disclosed herein may be configured to exploit the complementary/correlation property of the sinograms to complete a sinogram for each energy level. In some examples, the model can simultaneously generate both the low and high energy corrected sinograms through a multi-tasking, dual-channel output network. In other examples, two multi-scale interpolation models may be utilized, each configured for a single channel output, which takes in a (low or high energy) sinogram and is trained to output a corrected (low or high energy) sinogram.

In some examples, each sinogram that is input to the model(s) may be initialized in order to bring in complementary information from the other (different energy) sinogram using (a) direct non-adjusted data (e.g., where a missing view in a sinogram of one energy level is filled with the data of the complementary view in the sinogram of the other energy level), or by adjustment of the complementary data using (b) global statistics, (c) local information, (d) frequency or feature guidance, or a combination of more than one of these initializations.

Global statistics driven initialization scales the projection data from the complementary missing views based on low or high energy data statistics. In case of low energy data, the missing views are filled with a scaled version of the corresponding high energy data, computed using the statistics of the interpolating and target data. For example, if views 1-4 are missing from the low energy data, the missing views 1-4 may be filled in with scaled versions of the data from views 1-4 obtained at high energy. In the case of high energy data, the missing views are filled with a scaled version of the corresponding low energy data, also computed using the statistics of the interpolating and target data.

Local statistics driven initialization utilizes the concept that while there are similarities in attenuation between both the energy levels, they are not directly proportional and will change based on the material through which the X-rays are passing. This information is computed from the data and missing views in low energy are filled with a locally scaled/additive version of the high energy data. Likewise, the missing views in the high energy data are filled with locally scaled versions of the low energy data.

Another embodiment of the initialization is performed through feature level concatenation of the alternate energy data on the network. For example, feature representations may be generated of each sinogram and mixed to generate an initialization of each sinogram in the feature domain. This can be done within the network structure or the features can be generated using non-deep learning approach and then fed into the network.

The sinograms (whether initialized with data from the alternate sinogram or not) may be entered as input to a dual-channel output network, or each sinogram may be entered as input to a respective single-channel output network, as described above. The network(s) may be multi-scale networks trained to interpolate each missing view. Since the proposed architecture is designed to handle n gaps (n>1) in the missing views (and one missing view is a trivial solution for this architecture), a pyramid structure for multi-scale gradient optimization is implemented to ensure high precision at each scale of sinogram feature interpolation. The coarsest scale is defined by $n/X=1$, X is the down-sampling factor, to the finest scale of complete sinogram at highest resolution. In this way, features from each sinogram may be progressively downsampled by the multi-scale network to a lower resolution that includes, for each gap/set of missing views, only one missing view (e.g., rather than two, four, or more missing views as in the original sinogram). The downsampled features may be progressively upsampled back to the original resolution. The multi-scale network may be trained with a loss that is computed at each resolution level of the upsampled features. If there are $\ell$ resolution levels in the network, the loss is computed at each of the levels of resolution. For example, if the network is trained to fill in a sinogram having multiple sets of missing views, where each set of missing views includes four consecutive missing views, the network may be trained with three loss functions (a first loss function for the lowest resolution feature set with one missing and subsequently filled view, a second loss function for the middle resolution feature set with two missing and subsequently filled views, and a third loss function for the highest resolution feature set with four missing and subsequently filled views).

In some examples, the network may include a super-resolution network in the lower scale to recover the corrected sinogram. At the coarsest level, when one view is missing at the encoder end, a super-resolution network is trained to generate sinogram feature output at one level higher resolution. In this realization, the sinograms may not be initialized prior to entry to the network(s) or the sinograms may be initialized using feature level mixing.

In some examples, the network(s) may also use information from wavelet sub-band data at different frequency levels to boost the interpolation. The choice of frequency sub-bands may be driven by a similarity measure computed between the sinogram distribution for low and high energy data, which may be computed through metrics including structural similarity index measure (SSIM) and correlation from frequency contents in different sub-bands of low and high energy data. In some examples, the sub-band information using frequency domain decomposition can be used as a feature to the network. In another example, the sub-band information can be brought to same space as the sinogram. For example, sub-bands having higher similarity may be reconstructed and used as input to the network. This stage is optional and based on the performance, can be modified or removed.

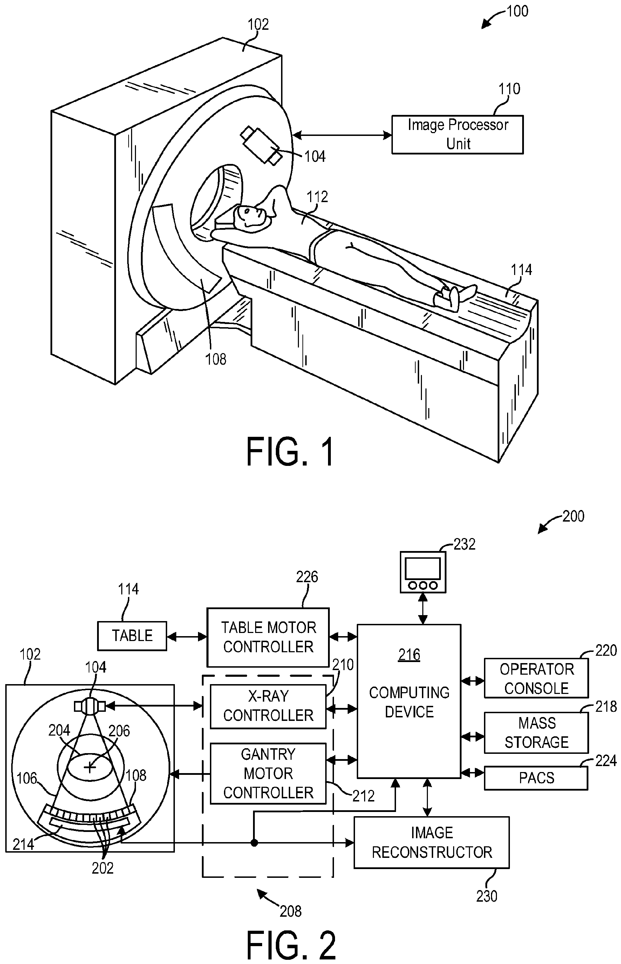

Embodiments of the present disclosure will now be described, by way of example, with reference to the figures. FIG. 1 illustrates an exemplary CT system 100 configured for CT imaging. Particularly, the CT system 100 is configured to image a subject 112 such as a patient, an inanimate object, one or more manufactured parts, and/or foreign objects such as dental implants, stents, and/or contrast agents present within the body. In one embodiment, the CT system 100 includes a gantry 102, which in turn, may further include at least one X-ray source 104 configured to project a beam of X-ray radiation 106 (see FIG. 2) for use in imaging the subject 112 laying on a table 114. Specifically, the X-ray source 104 is configured to project the X-ray radiation beams 106 towards a detector array 108 positioned on the opposite side of the gantry 102. Although FIG. 1 depicts only a single X-ray source 104, in certain embodiments, multiple X-ray sources and detectors may be employed to project a plurality of X-ray radiation beams 106 for acquiring projection data at different energy levels corresponding to the patient. In some embodiments, the X-ray source 104 may enable dual-energy gemstone spectral imaging (GSI) by rapid peak kilovoltage (kVp) switching. In some embodiments, the X-ray detector employed is a photon-counting detector which is capable of differentiating X-ray photons of different energies. In other embodiments, two sets of X-ray sources and detectors are used to generate dual-energy projections, with one set at low-kVp and the other at high-kVp. It should thus be appreciated that the methods described herein may be implemented with single energy acquisition techniques as well as dual energy acquisition techniques.

In certain embodiments, the CT system 100 further includes an image processor unit 110 configured to reconstruct images of a target volume of the subject 112 using an iterative or analytic image reconstruction method. For example, the image processor unit 110 may use an analytic image reconstruction approach such as filtered back projection (FBP) to reconstruct images of a target volume of the patient. As another example, the image processor unit 110 may use an iterative image reconstruction approach such as advanced statistical iterative reconstruction (ASIR), conjugate gradient (CG), maximum likelihood expectation maximization (MLEM), model-based iterative reconstruction (MBIR), and so on to reconstruct images of a target volume of the subject 112. As described further herein, in some examples the image processor unit 110 may use both an analytic image reconstruction approach such as FBP in addition to an iterative image reconstruction approach.

In some CT imaging system configurations, an X-ray source projects a cone-shaped X-ray radiation beam which is collimated to lie within an X-Y-Z plane of a Cartesian coordinate system and generally referred to as an "imaging plane." The X-ray radiation beam passes through an object being imaged, such as the patient or subject. The X-ray radiation beam, after being attenuated by the object, impinges upon an array of detector elements. The intensity of the attenuated X-ray radiation beam received at the detector array is dependent upon the attenuation of a radiation beam by the object. Each detector element of the array produces a separate electrical signal that is a measurement of the X-ray beam attenuation at the detector location. The attenuation measurements from all the detector elements are acquired separately to produce a transmission profile.

In some CT systems, the X-ray source and the detector array are rotated with a gantry within the imaging plane and around the object to be imaged such that an angle at which the radiation beam intersects the object constantly changes. A group of X-ray radiation attenuation measurements, e.g., projection data, from the detector array at one gantry angle is referred to as a "view." A "scan" of the object includes a set of views made at different gantry angles, or view angles, during one revolution of the X-ray source and detector. It is contemplated that the benefits of the methods described herein accrue to medical imaging modalities other than CT, so as used herein the term "view" is not limited to the use as described above with respect to projection data from one gantry angle. The term "view" is used to mean one data acquisition whenever there are multiple data acquisitions from different angles, whether from a CT, positron emission tomography (PET), or single-photon emission CT (SPECT) acquisition, and/or any other modality including modalities yet to be developed as well as combinations thereof in fused embodiments.

The projection data is processed to reconstruct an image that corresponds to a twodimensional slice taken through the object or, in some examples where the projection data includes multiple views or scans, a three-dimensional rendering of the object. One method for reconstructing an image from a set of projection data is referred to in the art as the filtered back projection technique. Transmission and emission tomography reconstruction techniques also include statistical iterative methods such as maximum likelihood expectation maximization (MLEM) and ordered-subsets expectation-reconstruction techniques as well as iterative reconstruction techniques. This process converts the attenuation measurements from a scan into integers called "CT numbers" or "Hounsfield units," which are used to control the brightness of a corresponding pixel on a display device.

To reduce the total scan time, a "helical" scan may be performed. To perform a "helical" scan, the patient is moved while the data for the prescribed number of slices is acquired. Such a system generates a single helix from a cone beam helical scan. The helix mapped out by the cone beam yields projection data from which images in each prescribed slice may be reconstructed.

As used herein, the phrase "reconstructing an image" is not intended to exclude embodiments of the present invention in which data representing an image is generated but a viewable image is not. Therefore, as used herein, the term "image" broadly refers to both viewable images and data representing a viewable image. However, many embodiments generate (or are configured to generate) at least one viewable image.

FIG. 2 illustrates an exemplary imaging system 200 similar to the CT system 100 of FIG. 1. In accordance with aspects of the present disclosure, the imaging system 200 is configured for imaging a subject 204 (e.g., the subject 112 of FIG. 1). In one embodiment, the imaging system 200 includes the detector array 108 (see FIG. 1). The detector array 108 further includes a plurality of detector elements 202 that together sense the X-ray radiation beam 106 (see FIG. 2) that pass through the subject 204 (such as a patient) to acquire corresponding projection data. Accordingly, in one embodiment, the detector array 108 is fabricated in a multi-slice configuration including the plurality of rows of cells or detector elements 202. In such a configuration, one or more additional rows of the detector elements 202 are arranged in a parallel configuration for acquiring the projection data.

In certain embodiments, the imaging system 200 is configured to traverse different angular positions around the subject 204 for acquiring desired projection data. Accordingly, the gantry 102 and the components mounted thereon may be configured to rotate about a center of rotation 206 for acquiring the projection data, for example, at different energy levels. Alternatively, in embodiments where a projection angle relative to the subject 204 varies as a function of time, the mounted components may be configured to move along a general curve rather than along a segment of a circle.

As the X-ray source 104 and the detector array 108 rotate, the detector array 108 collects data of the attenuated X-ray beams. The data collected by the detector array 108 undergoes pre-processing and calibration to condition the data to represent the line integrals of the attenuation coefficients of the scanned subject 204. The processed data are commonly called projections.

In some examples, the individual detectors or detector elements 202 of the detector array 108 may include photon-counting detectors which register the interactions of individual photons into one or more energy bins. It should be appreciated that the methods described herein may also be implemented with energy-integrating detectors.

The acquired sets of projection data may be used for basis material decomposition (BMD). During BMD, the measured projections are converted to a set of material-density projections. The material-density projections may be reconstructed to form a pair or a set of material-density map or image of each respective basis material, such as bone, soft tissue, and/or contrast agent maps. The density maps or images may be, in turn, associated to form a volume rendering of the basis material, for example, bone, soft tissue, and/or contrast agent, in the imaged volume.

Once reconstructed, the basis material image produced by the imaging system 200 reveals internal features of the subject 204, expressed in the densities of two basis materials. The density image may be displayed to show these features. In traditional approaches to diagnosis of medical conditions, such as disease states, and more generally of medical events, a radiologist or physician would consider a hard copy or display of the density image to discern characteristic features of interest. Such features might include lesions, vessels, sizes and shapes of particular anatomies or organs, and other features that would be discernable in the image based upon the skill and knowledge of the individual practitioner.

In one embodiment, the imaging system 200 includes a control mechanism 208 to control movement of the components such as rotation of the gantry 102 and the operation of the X-ray source 104. In certain embodiments, the control mechanism 208 further includes an X-ray controller 210 configured to provide power and timing signals to the X-ray source 104. Additionally, the control mechanism 208 includes a gantry motor controller 212 configured to control a rotational speed and/or position of the gantry 102 based on imaging requirements.

In certain embodiments, the control mechanism 208 further includes a data acquisition system (DAS) 214 configured to sample analog data received from the detector elements 202 and convert the analog data to digital signals for subsequent processing. The DAS 214 may be further configured to selectively aggregate analog data from a subset of the detector elements 202 into socalled macro-detectors, as described further herein. The data sampled and digitized by the DAS 214 is transmitted to a computer or computing device 216. In one example, the computing device 216 stores the data in a storage device 218. The storage device 218, for example, may include a hard disk drive, a floppy disk drive, a compact disk-read/write (CD-R/W) drive, a Digital Versatile Disc (DVD) drive, a flash drive, and/or a solid-state storage drive.

Additionally, the computing device 216 provides commands and parameters to one or more of the DAS 214, the X-ray controller 210, and the gantry motor controller 212 for controlling system operations such as data acquisition and/or processing. In certain embodiments, the computing device 216 controls system operations based on operator input. The computing device 216 receives the operator input, for example, including commands and/or scanning parameters via an operator console 220 operatively coupled to the computing device 216. The operator console 220 may include a keyboard (not shown), a mouse, and/or a touchscreen to allow the operator to specify the commands and/or scanning parameters.

Although FIG. 2 illustrates only one operator console 220, more than one operator console may be coupled to the imaging system 200, for example, for inputting or outputting system parameters, requesting examinations, plotting data, and/or viewing images. Further, in certain embodiments, the imaging system 200 may be coupled to multiple displays, printers, workstations, and/or similar devices located either locally or remotely, for example, within an institution or hospital, or in an entirely different location via one or more configurable wired and/or wireless networks such as the Internet and/or virtual private networks, wireless telephone networks, wireless local area networks, wired local area networks, wireless wide area networks, wired wide area networks, etc.

In one embodiment, for example, the imaging system 200 either includes, or is coupled to, a picture archiving and communications system (PACS) 224. In an exemplary implementation, the PACS 224 is further coupled to a remote system such as a radiology department information system, hospital information system, and/or to an internal or external network (not shown) to allow operators at different locations to supply commands and parameters and/or gain access to the image data.

The computing device 216 uses the operator-supplied and/or system-defined commands and parameters to operate a table motor controller 226, which in turn, may control a table 114 which may be a motorized table. Specifically, the table motor controller 226 may move the table 114 for appropriately positioning the subject 204 in the gantry 102 for acquiring projection data corresponding to the target volume of the subject 204.

As previously noted, the DAS 214 samples and digitizes the projection data acquired by the detector elements 202. Subsequently, an image reconstructor 230 uses the sampled and digitized X-ray data to perform high-speed reconstruction. Although FIG. 2 illustrates the image reconstructor 230 as a separate entity, in certain embodiments, the image reconstructor 230 may form part of the computing device 216. Alternatively, the image reconstructor 230 may be absent from the imaging system 200 and instead the computing device 216 may perform one or more functions of the image reconstructor 230. Moreover, the image reconstructor 230 may be located locally or remotely, and may be operatively connected to the imaging system 200 using a wired or wireless network. Particularly, one exemplary embodiment may use computing resources in a "cloud" network cluster for the image reconstructor 230.

In one embodiment, the image reconstructor 230 stores the images reconstructed in the storage device 218. Alternatively, the image reconstructor 230 may transmit the reconstructed images to the computing device 216 for generating useful patient information for diagnosis and evaluation. In certain embodiments, the computing device 216 may transmit the reconstructed images and/or the patient information to a display or display device 232 communicatively coupled to the computing device 216 and/or the image reconstructor 230. In some embodiments, the reconstructed images may be transmitted from the computing device 216 or the image reconstructor 230 to the storage device 218 for short-term or long-term storage.

The various methods and processes described further herein may be stored as executable instructions in non-transitory memory on a computing device (or controller) in imaging system 200, such as computing device 216 and/or image reconstructor 230, where the instructions are executable via one or more processors to carry out the methods and processes described herein. In some embodiments, computing device 216 may include the instructions in non-transitory memory, and may apply the methods described herein (via one or more processors), at least in part, to projection data (e.g., sinograms) in order to fill in missing views before the projection data is reconstructed via image reconstructor 230. In another embodiment, the methods and processes described herein may be performed by image reconstructor 230. In a still further embodiment, the methods and processes described herein may be distributed across image reconstructor 230 and computing device 216.

In one embodiment, the display 232 allows the operator to evaluate the imaged anatomy after image reconstruction. The display 232 may also allow the operator to select a volume of interest (VOI) and/or request patient information, for example, via a graphical user interface (GUI) for a subsequent scan or processing, select a scan protocol for carrying out the scan, and the like.

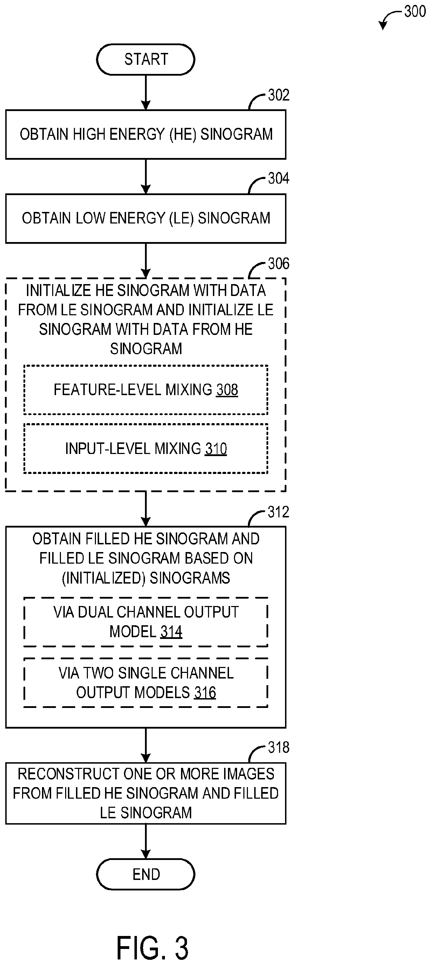

FIG. 3 is a flow chart illustrating a method 300 for filling sinograms obtained at different X-ray source energy levels. The method 300 will be described with relation to the systems depicted in FIGS. 1-2, but it should be understood that similar methods may be used with other systems without departing from the scope of this disclosure. Method 300 may be performed according to instructions stored in memory of a computing device operatively coupled to an imaging system, such as computing device 216, image reconstructor 230, a cloud-based computing system, or another suitable computing device. The imaging system may be a CT imaging system, as described above with respect to FIGS. 1-2, or another suitable imaging system capable of carrying out dual-energy imaging.

At 302, a high energy (HE) sinogram of an imaging subject is obtained. The HE sinogram may be generated by the imaging system and may be a visual representation of projection data (e.g., detector data) obtained when operating the imaging system X-ray source at a first, higher energy level (such as within a range of 120-140 kVp). At 304, a low energy (LE) sinogram of the imaging subject is obtained. The LE sinogram may be generated by the imaging system and may be a visual representation of projection data (e.g., detector data) obtained when operating the imaging system X-ray source at a second, higher energy level (such as within a range of 70-80 kVp). The HE and LE sinograms may be obtained during the same scan of the imaging subject, with the X-ray source controlled to alternate energy levels, such that a first set of views is obtained while the X-ray source is operated at the higher energy level, a second set of views is obtained while the X-ray source is operated at the lower energy level, a third set of views is obtained at the higher energy level, a fourth set of views is obtained at the lower energy level, etc. Each set of views may include two or more consecutive views. For example, the X-ray source may be controlled to switch energy levels every fourth view, such that the first set of views includes four consecutive views obtained at the higher energy level, the second set of views includes four consecutive views obtained at the lower energy level, and so forth, until all the views have been obtained and the scan is complete. In this way, each view may be obtained at only one energy level and each sinogram may be missing half the total views obtained during the scan. As used herein, missing views in a sinogram of a given energy level may refer to views not obtained at the given energy level but obtained at the other energy level. Using the example presented above, the first set of views may be obtained while the X-ray source is operated at the higher energy level and the second set of views may be obtained while the X-ray source is operated at the lower energy level. The resultant HE sinogram may include the first set of views and may be missing the second set of views. The resultant LE sinogram may be missing the first set of views and may include the second set of views.

At 306, method 300 optionally includes initializing the HE sinogram with data from the LE sinogram to create an initialized HE sinogram, and initializing the LE sinogram with data from the HE sinogram to create an initialized LE sinogram. The initializing of the HE sinogram may include adding data from the LE sinogram to the missing views of the HE sinogram, and specifically adding data from the complementary views of the LE sinogram to the missing views of the HE sinogram. For example, data from the second set of views of the LE sinogram (obtained while the X-ray source is operated at the lower energy level) may be added to the HE sinogram at a location where the second set of views would be located in the HE sinogram (had they been obtained for the HE sinogram), e.g., the missing second set views of the HE sinogram. Likewise, the initializing of the LE sinogram may include adding data from the HE sinogram to the missing views of the LE sinogram, and specifically adding data from the complementary views of the HE sinogram to the missing views of the LE sinogram. For example, data from the first set of views of the HE sinogram (obtained while the X-ray source is operated at the higher energy level) may be added to the LE sinogram at a location where the first set of views would be located in the LE sinogram (had they been obtained for the LE sinogram), e.g., the missing first set views of the LE sinogram.

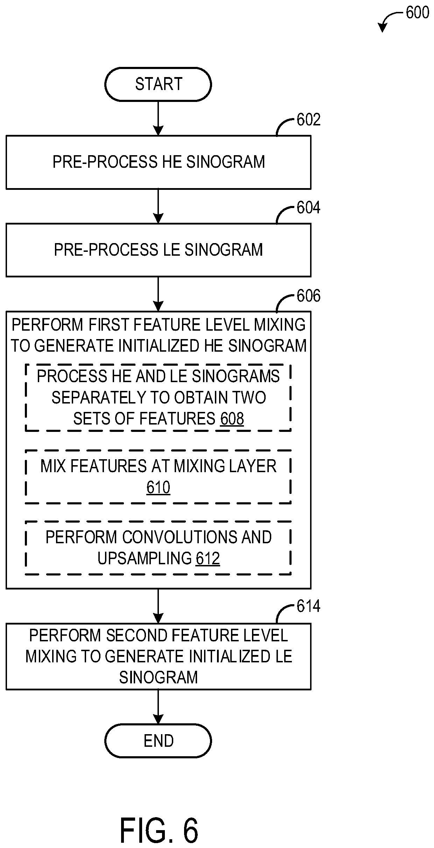

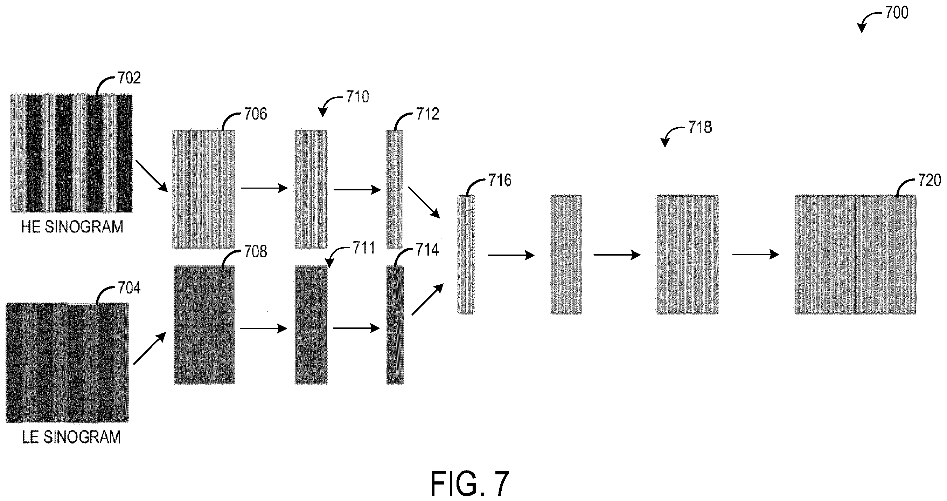

In some examples, the HE sinogram and the LE sinogram may be initialized using feature-level mixing, as indicated at 308. Additional details regarding the feature-level mixing are provided below with respect to FIGS. 6 and 7. Briefly, the feature-level mixing may include separately downsampling features of the obtained views of the HE sinogram and features of the obtained views of the LE sinogram to generate a set of HE sinogram features and a set of LE sinogram features, mixing the sets of features, and then upsampling the mixed features to create an initialized sinogram. The feature-level mixing may be performed for each sinogram separately using one or more deep learning models trained to output mixed features for each sinogram. For example, the deep learning model may be trained to mix the features from the LE sinogram with the HE sinogram to produce an initialized HE sinogram where the features from the LE sinogram are selected to fill in the missing features of the HE sinogram. Additionally, the deep learning model (or a separate deep learning model) may be trained to mix the features from the HE sinogram with the LE sinogram to produce an initialized LE sinogram where the features from the HE sinogram are selected to fill in the missing features of the LE sinogram.

In some examples, the HE sinogram and the LE sinogram may be initialized using input-level mixing, as indicated at 310. Input-level mixing may include direct sinogram data initialization, where all the data from the complementary views are added to the respective missing views. For example, all of the data from the second set of views of the LE sinogram may be added to the HE sinogram (at the location of the missing second set of views of the HE sinogram, e.g., between the first set of views and the third set of views) and all the data of the first set of views of the HE sinogram may be added to the LE sinogram (at the location of the missing first set of views of the LE sinogram, before the second set of views).

In other examples, the input-level mixing may include scaling the sinogram data that is added to the missing views. For example, the data from the second set of views of the LE sinogram may be scaled and the scaled data may be added to the HE sinogram (at the location of the missing second set of views of the HE sinogram, e.g., between the first set of views and the third set of views). Likewise, the data of the first set of views of the HE sinogram may be scaled and the scaled data added to the LE sinogram (at the location of the missing first set of views of the LE sinogram, before the second set of views). The sinogram data may be scaled globally or locally. The global scaling may include scaling the data from each view in the same manner, e.g., the data from each view may be reduced or increased by a scaling factor. The scaling factor may be determined from global statistics of the HE and/or LE sinograms. For example, when initializing the HE sinogram, the scaling factor may be based on a maximum value of the HE sinogram. In another example, the scaling factor may be based on a ratio of values in the HE sinogram to values in the LE sinogram (e.g., maximum values, average values, etc.). The local scaling may include scaling the data from a given view or views differently than one or more other views based on the material composition of the imaging subject (and hence the material that the X-rays passed through before reaching the detector). To perform the local scaling, a moving average of intensity values may be calculated for the HE sinogram and the LE sinogram independently over pre-defined averaging windows. For example, a first average intensity of the HE sinogram and a second average intensity of the LE sinogram may be calculated for the first 20 views; a third average intensity of the HE sinogram and a fourth average intensity of the LE sinogram may be calculated for the next 20 views; etc. When initializing the HE sinogram, a first ratio is computed of the average intensity of the HE sinogram and the average intensity of the LE sinogram over each averaging window (e.g., for the first 20 views, the first ratio may be of the first average intensity and the second average intensity described above), and the complementary data from the LE sinogram is scaled based on the respective first ratio for the data in each averaging window. When initializing the LE sinogram, a second ratio is computed of the average intensity of the LE sinogram and the average intensity of the HE sinogram over each averaging window (e.g., for the first 20 views, the second ratio may be of the second average intensity and the first average intensity described above), and the complementary data from the HE sinogram is scaled based on the respective second ratio for the data in each averaging window.

At 312, method 300 includes obtaining a filled HE sinogram and a filled LE sinogram based on the HE and LE sinograms (which may or may not be initialized). When utilized, the initialized sinograms may include the HE and LE sinograms that were initialized using input-level mixing as described above, or the initialized sinograms may include the mixed features of the HE and LE sinograms obtained via the feature-level mixing described above. The filled HE sinogram may be obtained by entering the (initialized) HE sinogram as input to a multi-scale interpolation model trained to output the filled HE sinogram, whereby the multi-scale interpolation model performs a progressive (e.g., multi-scale) interpolation of the missing views using the neighboring views of the HE sinogram and optionally the added data from the LE sinogram. The filled LE sinogram may be obtained by entering the (initialized) LE sinogram as input to a multi-scale interpolation model trained to output the filled LE sinogram, whereby the multi-scale interpolation model performs a progressive (e.g., multi-scale) interpolation of the missing views using the neighboring views of the LE sinogram and optionally the added data from the HE sinogram.

The filled HE sinogram and the filled LE sinogram may be obtained via the same multi-scale interpolation model, which may be a dual-channel output model, as indicated at 314. In the dual-channel output model, the (initialized) HE sinogram and the (initialized) LE sinogram may be entered as input to the dual-channel output model, and the dual-channel output model may output the filled HE sinogram on one channel and the filled LE channel on the other channel. However, in other examples, the filled HE sinogram and the filled LE sinogram may be obtained via two separate single channel output models, as indicated at 316. In such an example, the (initialized) HE sinogram may be entered as input to a first multi-scale interpolation model trained to output the filled HE sinogram, and the (initialized) LE sinogram may be entered as input to a second multi-scale interpolation model trained to output the filled LE sinogram. Additional details about obtaining the filled HE and LE sinograms and the multi-scale interpolation model(s) are presented below with respect to FIGS. 3,4, 5, and 8-13.

At 318, one or more images are reconstructed from the filled HE sinogram and the filled LE sinogram. The one or more images may be virtual monoenergetic images and/or basis material images reconstructed according to a suitable reconstruction technique. For example, an HE image may be reconstructed from the filled HE sinogram using filtered backprojection and an LE image may be reconstructed from the filled LE sinogram using filtered backprojection. A virtual monoenergetic image may be formed by a linear or non-linear combination of the HE image and the LE image. At least in some examples, the one or more images may be reconstructed directly from the filled HE sinogram and the filled LE sinogram, without performing any additional processing on the filled HE sinogram and the filled LE sinogram prior to reconstruction. The one or more images may be displayed on a display device and/or stored in memory (e.g., as part of a patient exam). Method 300 then returns.

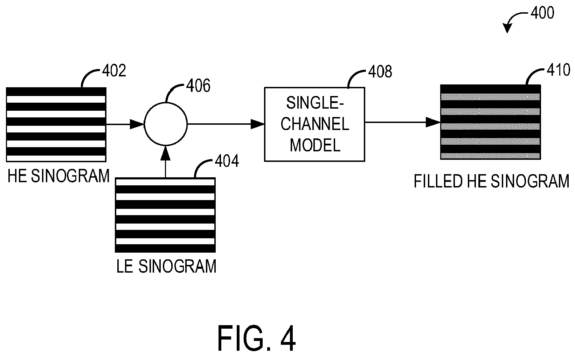

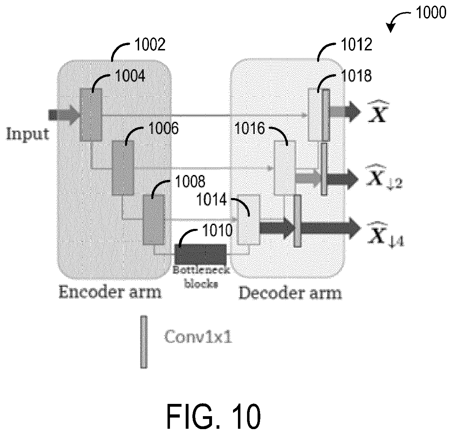

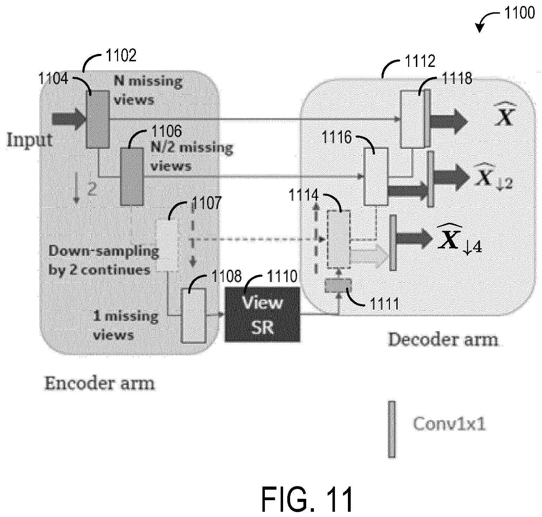

FIG. 4 schematically shows an example process 400 for obtaining a filled HE sinogram using a single-channel output model. The process 400 includes initializing an HE sinogram 402 using data from an LE sinogram 404 at an initialization block 406. The HE sinogram 402 may be missing views that are present in the LE sinogram 404, which is schematically shown in FIG. 4. For example, each sinogram may include channel data (e.g., detector output values, such as HU values) plotted as a function of view and the black lines may indicate obtained sets of views while the white lines may indicate missing sets of views. It is to be appreciated that in imaging systems that include multi-row detectors, multiple HE and LE sinograms may be generated, one set of HE and LE sinograms for each row of detectors. The processes described herein apply to matched sinograms, e.g., an HE sinogram generated from detector data from one row of the detector and a corresponding LE sinogram generated from detector data from the same one row of the detector, and that the processes may be applied to each matched set of HE/LE sinograms corresponding to each row of the detector. The HE sinogram may be initialized as explained above with respect to FIG. 3, e.g., via feature-level mixing or at the input level. The initialized HE sinogram may be entered as input to a single-channel output model 408 trained to output a filled HE sinogram 410 based on the initialized HE sinogram. Example network architectures for the single-channel output model 408 are presented below with respect to FIGS. 10 and 11. While not shown in FIG. 4, it is to be appreciated that a similar process would be carried out for obtaining a filled LE sinogram, whereby an initialized LE sinogram may be generated from the HE sinogram 402 and the LE sinogram 404, and the initialized LE sinogram may be entered as input to a second single-channel output model, which is trained to output a filled LE sinogram based on the initialized LE sinogram. Further, in some examples, the HE sinogram 402 may not be initialized prior to entry to the single-channel output model 408, as will be explained in more detail below with respect to FIG. 11.

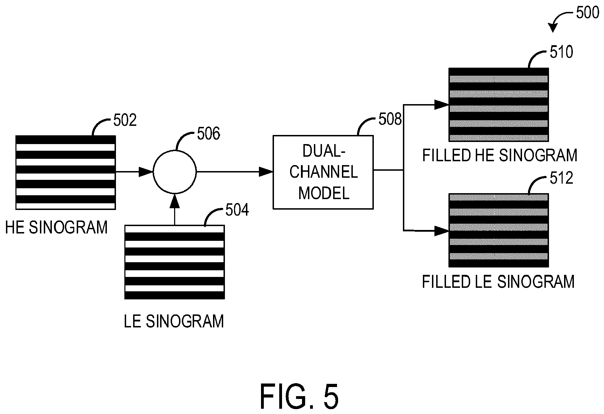

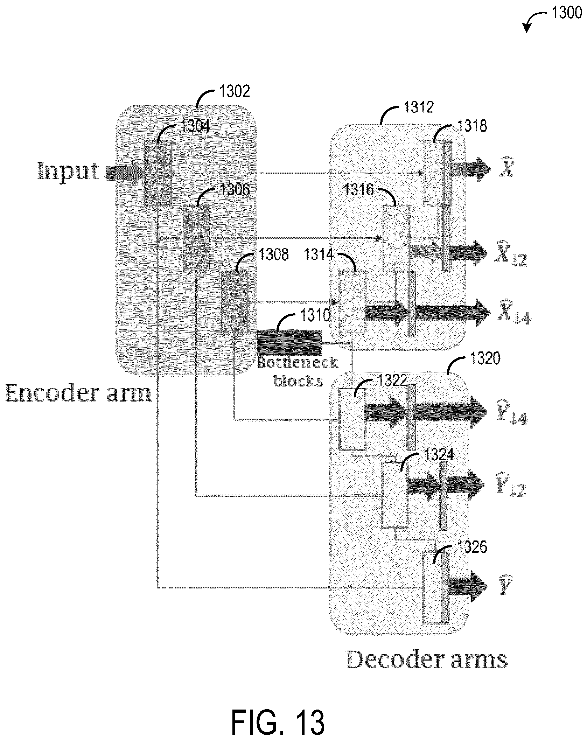

FIG. 5 schematically shows an example process 500 for obtaining a filled HE sinogram and a filled LE sinogram using a dual-channel output model. The process 500 includes initializing an HE sinogram 502 using data from an LE sinogram 504 at an initialization block 506, and also initializing the LE sinogram 504 from the HE sinogram 502 at the initialization block 506. The HE sinogram 502 may be same as the HE sinogram 402 and the LE sinogram 504 may be the same as the LE sinogram 404. The HE sinogram and the LE sinogram may be initialized as explained above with respect to FIG. 3, e.g., via feature-level mixing or at the input level. Each of the initialized HE sinogram and the initialized LE sinogram may be entered as input to a dual-channel output model 508 trained to output a filled HE sinogram 510 based on the initialized HE sinogram and a filled LE sinogram 512 based on the initialized LE sinogram. An example network architecture for the dual-channel output model 508 is presented below with respect to FIG. 13. Further, in some examples, the HE sinogram 502 and LE sinogram 504 may not be initialized prior to entry to the dual-channel output model 508, as will be explained in more detail below with respect to FIG. 11.

FIG. 6 is a flow chart illustrating a method for performing feature-level mixing of HE sinogram and LE sinogram features. Method 600 may be performed according to instructions stored in memory of a computing device operatively coupled to an imaging system, such as computing device 216, image reconstructor 230, a cloud-based computing system, or another suitable computing device. In some examples, method 600 may be carried out as part of method 300, for example method 600 may be performed at the feature-level mixing 308 of method 300.

At 602, method 600 includes pre-processing an HE sinogram. The HE sinogram may be the HE sinogram described above with respect to FIG. 3 and/or the HE sinogram of FIG. 4 or 5. The pre-processing may include discarding the missing views in the HE sinogram, to generate a partial HE sinogram including only the obtained views. Likewise, at 604, method 600 includes pre-processing an LE sinogram. The LE sinogram may be the LE sinogram described above with respect to FIG. 3 and/or the LE sinogram of FIG. 5. The pre-processing may include discarding the missing views in the LE sinogram, to generate a partial LE sinogram including only the obtained views.

At 606, method 600 includes performing a first feature level mixing to generate an initialized HE sinogram. The first feature level mixing may be performed by a first feature mixer, which may include convolutional layers of the multi-scale interpolation model (e.g., the single-channel output model 408 or the dual-channel output model 508), or the first feature mixer may be separate from the multi-scale interpolation model. Performing the first feature level mixing may include processing the pre-processed partial HE sinogram and partial LE sinogram separately to generate two sets of features, as indicated at 608. For example, the partial HE sinogram may be passed through multiple convolutional layers of the first feature mixer until a target downsampled HE sinogram feature set is reached, which may comprise a first set of HE sinogram features. In parallel, the partial LE sinogram may be passed through multiple convolutional layers of the first feature mixer until a target downsampled LE sinogram feature set is reached, which may comprise a second set of LE sinogram features. Performing the first feature level mixing may further include mixing features at a mixing layer of the first feature mixer, as indicated at 610. For example, the first set of HE sinogram features may be mixed with the second set of LE sinogram features. The mixing may include adding the first set of HE sinogram features and the second set of LE sinogram features, interleaving the first set of HE sinogram features with the second set of LE sinogram features, or another suitable method for combining the first and second sets of features. Performing the first feature level mixing may additionally include performing convolutions and upsampling the mixed features, as indicated at 612, until the original resolution of the HE sinogram is reached. The mixed first and second set of features may be passed through multiple convolutional layers of the first feature mixer in order to upsample the mixed features back to the original resolution (e.g., number of views) of the HE sinogram, to thereby create the initialized HE sinogram.

At 614, method 600 includes performing a second feature level mixing to generate an initialized LE sinogram. The second feature level mixing may be performed by a second feature mixer in a manner similar to the first feature level mixing. The second feature mixer may be included as part of the multi-scale interpolation model trained to fill the LE sinogram, or the second feature mixer may be separate from the multi-scale interpolation model. To perform the second feature level mixing, the partial HE sinogram and the partial LE sinogram may be processed as described above, e.g., passed though convolutional layers of the second feature mixer in parallel to generate a third set of HE sinogram features and a fourth set of LE sinogram features, the third and fourth sets of sinogram features may be mixed at a mixing layer of the second feature mixer, and the mixed features may be passed through multiple convolutional layers of the second feature mixer to upsample the mixed features back to the original resolution of the LE sinogram.

In this way, initializing the HE sinogram may include processing the HE sinogram through a first set of operations and downsampling (e.g., the first set of operations and downsampling may include the pre-processing to discard the missing views, the feature extraction, convolutions, poolings, and/or downsampling) and to generate a first set of features, which may be higher-dimensional features corresponding the HE sinogram. The LE sinogram may also be processed through a second set of operations and downsampling to generate a second set of features including higher-dimensional features corresponding the LE sinogram. The first set of features and the second set of features may be mixed to form a mixed set of features and the mixed set of features may be upsampled to form the initialized HE sinogram. A similar process may be performed to initialize the LE sinogram.

The first feature mixer and the second feature mixer may be separate mixers that are trained separately in a manner that facilitates appropriate selection of features from the LE sinogram to add to the HE sinogram and features from the HE sinogram to add to the LE sinogram, respectively. For example, the first feature mixer may be trained with a loss function calculated based on a difference between the initialized HE sinogram output by the first feature mixer and a ground truth HE sinogram that includes a complete set of views (e.g., all possible views the imaging system is capable of obtaining are obtained while the X-ray source of the imaging system is operated at the higher energy level). During training, the input to the first feature mixer may include a culled HE sinogram, where multiple sets of views of the ground truth HE sinogram are removed to mimic an HE sinogram obtained with the imaging system operating in the dual-energy mode (and thus missing half of the total views). Similarly, the second feature mixer may be trained with a loss function calculated based on a difference between the initialized LE sinogram output by the second feature mixer and a ground truth LE sinogram that includes a complete set of views (e.g., all possible views the imaging system is capable of obtaining are obtained while the X-ray source of the imaging system is operated at the lower energy level). During training, the input to the first feature mixer may include a culled LE sinogram, where multiple sets of views of the ground truth LE sinogram are removed to mimic an LE sinogram obtained with the imaging system operating in the dual-energy mode (and thus missing half of the total views). Accordingly, the features extracted by the first feature mixer and the second feature mixer may be different, e.g., the first set of features of the HE sinogram may be different than the third set of features of the HE sinogram and the second set of features of the LE sinogram may be different than the fourth set of features of the LE sinogram. In this way, despite starting with the same partial HE and LE sinograms, the first feature level mixer may output a different initialized sinogram than the initialized sinogram output by the second feature level mixer.

FIG. 7 schematically shows an example process 700 for generating an initialized HE sinogram using feature level mixing. The feature level mixing illustrated by process 700 may be performed by the first feature level mixer according to method 600 described above. Process 700 starts with an HE sinogram 702 and an LE sinogram 704, which are obtained during a scan of an imaging subject using an imaging system (e.g., imaging system 200) in a dual-energy mode, as explained previously with respect to FIGS. 3-5. In the example shown in FIG. 7, the missing views are illustrated in the darker color and the obtained views are illustrated in the lighter color. The HE sinogram 702 is pre-processed to remove the missing views to generate a partial HE sinogram 706. Likewise, the LE sinogram is pre-processed to remove the missing views to generate a partial LE sinogram 708.

The partial HE sinogram 706 is passed through multiple convolutional layers 710 with pooling to generate a first set of HE sinogram features 712. The first set of HE sinogram features 712 may be at a lower resolution than the original HE sinogram 702 and the partial HE sinogram 706. For example, if the original HE sinogram includes 32 views (16 of which are missing views) and the partial HE sinogram includes 16 views, the first set of HE sinogram features may have four views. The partial LE sinogram 708 is passed through multiple convolutional layers 711 with pooling to generate a second set of LE sinogram features 714 at the same resolution as the first set of HE sinogram features.

The first set of HE sinogram features 712 and the second set of LE sinogram features 714 are mixed to create a set of mixed features 716. The mixing may include adding, interleaving, or another suitable mechanism to mix the two sets of features. In the example shown, the second set of LE sinogram features 714 is added to the first set of HE sinogram features 712 (e.g., the first view of the first set of LE sinogram features is added to/concatenated with the first view of the second set of HE sinogram features, etc.). The set of mixed features 716 is passed through multiple convolutional layers 718 with upsampling until an initialized HE sinogram 720 is generated of the same resolution as the original HE sinogram (e.g., 32 views in this example). In doing so, the missing views of the original HE sinogram 702 are filled with information from the obtained views of the LE sinogram 704.

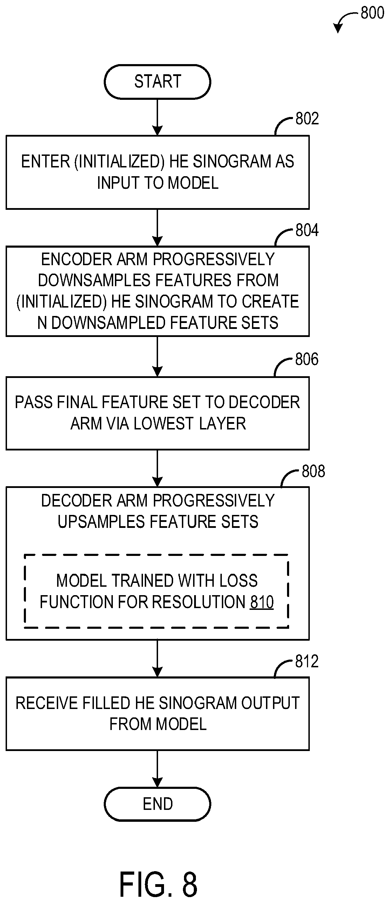

FIG. 8 is a flow chart illustrating a method 800 for obtaining a filled HE sinogram from an HE sinogram with missing views using a single-channel output multi-scale interpolation model. Method 800 may be performed according to instructions stored in memory of a computing device operatively coupled to an imaging system, such as computing device 216, image reconstructor 230, a cloud-based computing system, or another suitable computing device. In some examples, method 800 may be carried out as part of method 300, for example method 800 may be performed at 312 of method 300. Method 800 specifically illustrates the process for obtaining a filled HE sinogram, but it is to be appreciated that the process of obtaining a filled LE sinogram may be carried out identically to method 800, starting with an LE sinogram missing views rather than an HE sinogram and using a multi-scale interpolation model specifically trained to fill LE sinograms.

At 802, an HE sinogram is entered as input to a multi-scale interpolation model. The HE sinogram may be initialized with data from the complementary LE sinogram in some examples. The initialized HE sinogram may be created as explained above with respect to FIG. 3, e.g., by filling in missing views of an original HE sinogram with data from complementary views of an original LE sinogram using feature-level mixing or input-level mixing. The original HE sinogram may be missing views, where the missing views are grouped into sets of consecutive missing views interleaved with sets of obtained views. Each set of missing views may include at least two consecutive missing views. The multi-scale interpolation model may be a single-channel output model, such as single-channel output model 408, trained to only output a filled HE sinogram.

The multi-scale interpolation model may have an encoder-decoder architecture with the encoder arm configured to progressively process (e.g., downsample or otherwise decrease a resolution of) extracted features from the HE sinogram to a lower resolution where, if the features were to be mapped back to the sinogram domain, each set of missing (though optionally initialized with LE sinogram data in the initialized HE sinogram) views includes only one missing view. For example, if the original HE sinogram includes alternating sets of four consecutive missing views and four obtained views, the progressive processing may include a first processing where, if the processed features were to be mapped back to the sinogram domain, each set of missing/initialized views includes two missing/initialized views (rather than four) and each set of obtained views includes two obtained views. The progressive processing may then further include a second processing where, if the processed features were to be mapped back to the sinogram domain, each set of two missing/initialized views is processed (e.g., downsampled) to one missing/initialized view (and each set of two obtained views is processed/downsampled to one obtained view). The multi-scale interpolation model may then interpolate features of each missing/initialized view, which may be more accurate and faster than attempting to interpolate features of multiple consecutive missing/initialized views.

Thus, at 804, the encoder arm of the multi-scale interpolation model progressively downsamples extracted features of the HE sinogram to create N downsampled feature sets. The number N of downsampled feature sets that are created may depend on how many views are missing in each set of missing views of the HE sinogram. If two views are missing in each set, one downsampled feature set is created. If four views are missing in each set, two downsampled feature sets are created. If eight views are missing in each set, three downsampled feature sets are created. The downsampling may be in the view direction, such that at each round of downsampling, two views are downsampled to one view. However, in some examples, the downsampling may also occur in the channel direction. The downsampled feature set that, if mapped back to the sinogram domain, were to include only one missing/initialized view per original set of missing views (alternating with one downsampled obtained view) may be referred to as the final downsampled feature set.

At 806, the final downsampled feature set is passed to a decoder arm of the multi-scale interpolation model via a lowest layer of the multi-scale interpolation model, which may be a bottleneck layer or a super resolution layer. The decoder arm of the multi-scale interpolation model may progressively process the feature sets from the encoder arm in order to increase a spatial resolution of the feature sets from the encoder arm until a final expanded feature set is formed. The processing performed in the decoder arm may include upsampling, for example. Thus, the decoder arm of the multi-scale interpolation model progressively upsamples the downsampled feature sets, as indicated at 808, with the model trained with a respective loss function for each resolution/stage of processing in the decoder arm (as indicated at 810) until an original resolution (e.g., matching the resolution of the original HE sinogram) feature set is formed, which is then passed through an output layer to form the filled HE sinogram that is output from the model, as indicated at 812.

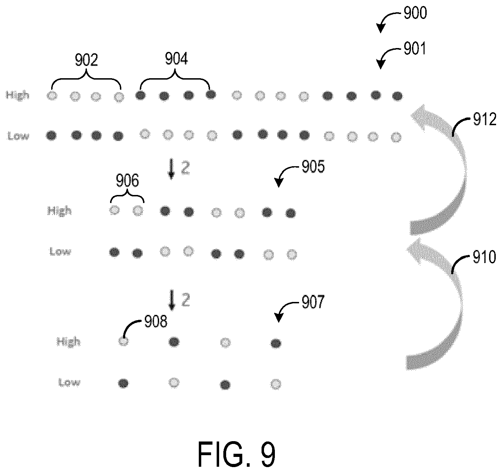

FIG. 9 schematically shows an example 900 of the progressive downsampling and upsampling that occurs in the multi-scale interpolation model(s) described herein. A plurality of views 901 from each of an HE sinogram and an LE sinogram are schematically depicted, with the views from the HE sinogram labeled as "High" in FIG. 9 and the views from the LE sinogram labeled as "Low." The obtained views are depicted as the lighter color circles while the missing views are depicted as the darker color circles. For example, the HE sinogram includes a first set of obtained views 902, which includes four consecutive obtained views. The HE sinogram also includes a second set of missing views 904, which includes four consecutive missing views. The obtained and missing sets of views alternate in a similar manner for the entirety of the HE sinogram. Each view may represent a row of pixels in the respective sinogram, such that each view of the first set of obtained views 902 and each view of the second set of missing views 904 represents a respective row of pixels of the HE sinogram. The obtained views may include intensity values (e.g., HU values) for each pixel, while the missing views may include no values (e.g., a value of 0) for each pixel.

The LE sinogram includes complementary missing and obtained views, such that the LE sinogram includes missing views where the HE sinogram includes obtained views and the LE sinogram includes obtained views where the HE sinogram includes missing views. Each view of the LE sinogram shown in FIG. 9 may represent a row of pixels in the LE sinogram, as explained above for the HE sinogram. The obtained views may include intensity values (e.g., HU values) for each pixel, while the missing views may include no values (e.g., a value of 0) for each pixel.

During initialization, when performed, the missing views of the HE sinogram (e.g., the second set of missing views 904) are initialized with information from the respective complementary views of the LE sinogram and the missing views of the LE sinogram are initialized with information from the respective complementary views of the HE sinogram. As a specific example, the first view of the LE sinogram (which is a missing view) may be initialized with information from the first view of the HE sinogram. For example, scaled intensity values from the first row of pixels (represented by the first view) of the LE sinogram may be added to the first row of pixels of the HE sinogram.

The features from the plurality of views are extracted and a first round of downsampling is performed on the extracted features to form a second plurality of views 905. The downsampling is by a factor of 2, and as such the sets of obtained and missing/initialized views each include two consecutive views. For example, the first set of obtained views 902 is downsampled to a first downsampled set of obtained views 906, with the downsampling going from four views to two views. In this way, the features extracted from the input HE sinogram may be downsampled so that, if the downsampled features were mapped back to the sinogram domain, the four rows of pixels corresponding to the first set of obtained views 902 would be downsampled to two rows of pixels, and likewise for the rows of pixels corresponding to the missing views (e.g., downsampled from four rows to two rows). After another round of downsampling (again by a factor of 2), a third (e.g., final) plurality of views 907 is formed, with each set of views now only including one view. For example, the first downsampled set of obtained views 906 has been downsampled to a single first downsampled view 908, that, if mapped back to the sinogram domain, would represent one row of pixels. According, for both the HE and LE sinograms at the final downsampling, the missing views alternate with the obtained views one by one, such that if the features, after the final downsampling, were mapped back to sinogram space, each missing/initialized row of pixels would have one or two neighboring obtained rows of pixels. As explained previously, interpolating a single missing view based on one or two neighboring obtained views may be relatively easy to perform and accurate, compared to interpolating four consecutive views. Thus, each missing/initialized view may be interpolated during the progressive upsampling, as shown by arrows 910 and 912, to return to the original resolution/number of views as the initialized HE and LE sinograms.

FIG. 10 schematically shows a first example architecture 1000 for a single-channel output, multi-scale interpolation model. The multi-scale interpolation model according to the first architecture 1000 includes an encoder arm 1002 that takes as input a sinogram (e.g., an HE sinogram or an LE sinogram) having missing views. In some examples, the input sinogram may be an initialized sinogram (e.g., an initialized HE or LE sinogram). The encoder arm 1002 extracts a set of features from the input sinogram, which may be referred to as a first feature set 1004. The encoder arm 1002 downsamples the first feature set 1004 by a factor of two in the view direction to form an intermediate feature set 1006. The encoder arm 1002 downsamples the intermediate feature set 1006 by a factor of two in the view direction to form a final feature set 1008.

The final feature set 1008 is passed through a bottleneck layer 1010. The output from the bottleneck layer 1010 is passed to a decoder arm 1012 along with the final feature set 1008, where the output from the bottleneck layer is concatenated with the final feature set 1008 to form a first expanded feature set 1014. The decoder arm upsamples the first expanded feature set 1014 by a factor of two and concatenates the upsampled expanded feature set with the intermediate feature set 1006 to form an intermediate expanded feature set 1016. The decoder arm upsamples the intermediate expanded feature set 1016 by a factor of two and concatenates the upsampled expanded feature set with the first feature set 1004 to form a final expanded feature set 1018. The final expanded feature set 1018 is passed through an output layer (e.g., 1x1 convolution) to generate the filled sinogram ($\hat{X}$) that is output by the model.

The feature extraction, progressive processing to decrease the resolution (e.g., downsampling), and progressive processing to increase the resolution (e.g., upsampling) of the multi-scale interpolation model may be performed according to weights and biases of the multi-scale interpolation model that are set during training of the multi-scale interpolation model. Typically, the weights and biases may be set during training based on a loss function calculated between the final output of the model ($\hat{X}$) (e.g., the filled sinogram) and a corresponding ground truth sinogram ($X$). For example, the ground truth sinogram may be a sinogram where all possible views are obtained at one energy level, e.g., a ground truth HE sinogram would include all views obtained at the higher energy level and would not include any missing views.

However, the multi-scale interpolation model of the present disclosure is trained with multiple loss functions calculated at different stages (e.g., layers/resolutions) of the model. As shown in FIG. 10, during training, the first expanded feature set 1014 may be passed through a 1x1 convolutional output layer to form an output first interpolated sinogram ($\hat{X}_{\downarrow 4}$). Likewise, the intermediate expanded feature set 1016 may be passed through a 1x1 convolutional output layer to form an output intermediate interpolated sinogram ($\hat{X}_{\downarrow 2}$). Each sinogram output by the model (the output filled sinogram $X$, the output intermediate interpolated sinogram $\hat{X}_{\downarrow 2}$, and the output first interpolated sinogram $\hat{X}_{\downarrow 4}$) may be used along with a corresponding ground truth sinogram to calculate a respective loss function, and the model may be updated during training based on each loss function.

The overall loss for a round of training with N levels of downsampling may be calculated according to the following equation:

$$\mathcal{L} = \sum_{i=1}^{N} \lambda_i \mathcal{L}_i$$

The loss functions ($\mathcal{L}$) may be calculated according to the following equations:

$$\mathcal{L}_1 = \left\| \left( \hat{X} - X \right) \odot mask \right\|$$

$$\mathcal{L}_2 = \left\| \left( \hat{X}_{\downarrow 2} - X_{\downarrow 2} \right) \odot mask_{\downarrow 2} \right\|$$

$$\mathcal{L}_4 = \left\| \left( \hat{X}_{\downarrow 4} - X_{\downarrow 4} \right) \odot mask_{\downarrow 4} \right\|$$

The losses that are calculated may be mean absolute error (MAE)/SSIM/spectral loss or feature loss. Thus, a loss function is calculated for each sinogram (at different resolutions) output by the model based on a difference between the output sinogram and a respective ground truth sinogram. The downsampled ground truth sinograms for calculating the loss for the output first interpolated sinogram and the output intermediate interpolated sinogram ($X_{\downarrow 4}$ and $X_{\downarrow 2}$, respectively) may be generated by downsampling the ground truth sinogram ($X$). Further, a respective mask may be applied during each loss calculation so that the loss is calculated only on the missing/initialized views and the loss is not calculated on the obtained views, which may train the model to preserve the data in each obtained view.

Thus, the multi-scale interpolation model shown in FIG. 10 takes a sinogram having missing or initialized views as input. The sinogram values from the input sinogram are converted to features, of decreasing spatial resolution via progressive downsampling, and then these features are expanded, via progressive upsampling, into a complete sinogram where the missing or initialized views are filled with sinogram data.

At each spatial resolution, a sinogram mask at that resolution is generated, which is a matrix of values. A ground truth sinogram mask at the corresponding resolution, which is also a matrix of values, and which has been shrunk to the match the resolution/size of the sinogram mask, is subtracted from the sinogram mask and the difference is a matrix of differences.

Element-wise multiplication may be performed between the difference matrix and a mask, where the mask is also scaled to the appropriate resolution. The mask may have a value of 1 at each point corresponding to an interpolated value, and a value of 0 at each point corresponding to a non-interpolated value, so that only differences between ground truth sinogram intensity and interpolated sinogram intensity is used in the loss. However, in some examples, the mask could include other numbers, allowing to differentially weight interpolated versus non-interpolated regions, or just to arbitrarily weight parts of the sinogram, e.g., a region of interest which should be more highly emphasized by the loss.

The norm of the matrix is taken, which may include squaring each element, adding all the squared values up, and taking the square root of that sum, so that the loss a positive scaler value.

The above process is performed for each resolution level, and then the final loss is obtained as a weighted sum of the losses at each resolution level, as explained above. This allows for a differential amount of emphasis to be put on the "interpolation" results at each spatial resolution.

FIG. 11 schematically shows a second example architecture 1100 for a multi-scale interpolation model. The multi-scale interpolation model according to the second architecture 1100 is similar to the first architecture illustrated in FIG. 10 and thus includes an encoder arm 1102 that takes as input a sinogram with missing views (e.g., an HE or LE sinogram) and extracts a set of features from the sinogram, which may be referred to as a first feature set 1104. The encoder arm 1102 downsamples the first feature set 1104 by a factor of two in the view direction to form an intermediate feature set 1106. The encoder arm 1102 downsamples the intermediate feature set 1106 by a factor of two in the view direction to form a final feature set 1108. FIG. 11 also shows that more intermediate downsampling layers may be included (e.g., when the sets of missing views of the sinograms include eight consecutive missing views), such as to produce a second intermediate feature set 1107 prior to the formation of the final feature set.

Instead of relying on a bottleneck layer, the second architecture 1100 includes a super resolution layer 1110. The final feature set 1108 is passed through the super resolution layer 1110, which expands/upsamples the final feature set by a factor of two. The output 1111 from the super resolution layer 1110 is passed to a decoder arm 1112 along with the second intermediate feature set 1107, where the output 1111 is concatenated with the second intermediate feature set 1107 to form a first intermediate expanded feature set 1114. The decoder arm upsamples the first intermediate expanded feature set 1114 by a factor of two and concatenates the upsampled feature set with the intermediate feature set 1106 to form a second intermediate expanded feature set 1116. The decoder arm upsamples the second intermediate expanded feature set 1116 by a factor of two and concatenates the upsampled feature set with the first feature set 1104 to form a final expanded feature set 1118. The final expanded feature set 1118 is passed through an output layer (e.g., 1x1 convolution) to generate the filled sinogram ($\hat{X}$) that is output by the model. In examples where the second intermediate layer of downsampling is not performed, the output 1111 may be concatenated with the intermediate feature set 1106 to form the second intermediate expanded feature set 1116.

The multi-scale interpolation model according to the second architecture 1100 may be trained in the same way as the model according to the first architecture 1000, but may include one fewer loss function. While FIG. 11 shows three outputs with which loss functions may be calculated (the output filled sinogram $X$, the output intermediate interpolated sinogram $\hat{X}_{\downarrow 2}$, and the output first interpolated sinogram $\hat{X}_{\downarrow 4}$), the second architecture includes an additional (optional) downsampling layer. If the additional downsampling layer were omitted so that 1107 and 1114 were not formed, there would not be an output first interpolated sinogram $\hat{X}_{\downarrow 4}$ and the only outputs of the second architecture 1100 would be the output filled sinogram $X$ and the output intermediate upsampled sinogram $\hat{X}_{\downarrow 2}$.

The inclusion of the super resolution layer demands that the input to the super resolution layer has at least x/2 data and after the operation of the super-resolution layer, the output data (e.g., output from the super resolution layer) will be of size x. This will be available only at the level of the encoder where there is one alternate missing views, so that the total number of measurements are x/2. The missing views are removed to get the x/2 sized data. After super-resolution, an 'x' sized sinogram is output. Thus, the architecture 1100, with the super resolution layer, cannot utilize sinograms that have been initialized at the input layer using global or local scaling and only non-initialized or feature-level initialized sinograms may be used as input.

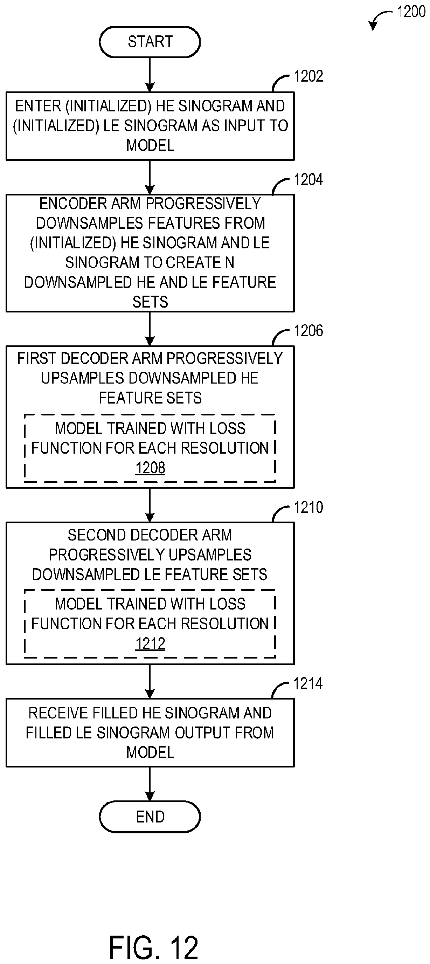

FIG. 12 is a flow chart illustrating a method 1200 for obtaining a filled HE sinogram from an HE sinogram with missing views and a filled LE sinogram from an LE sinogram with missing views using a dual-channel output multi-scale interpolation model. Method 1200 may be performed according to instructions stored in memory of a computing device operatively coupled to an imaging system, such as computing device 216, image reconstructor 230, a cloud-based computing system, or another suitable computing device. In some examples, method 1200 may be carried out as part of method 300, for example method 1200 may be performed at 312 of method 300.

At 1202, an HE sinogram and an LE sinogram are each entered as input to a multi-scale interpolation model. The HE sinogram may be initialized with data from the complementary LE sinogram, in some examples. The LE sinogram may be initialized with data from the complementary HE sinogram, in some examples. The initialized HE sinogram and initialized LE sinogram may be created as explained above with respect to FIG. 3, e.g., by filling in missing views of an original HE (or LE) sinogram with data from complementary views of an original LE (or HE) sinogram using feature-level mixing or input-level mixing. The original HE and LE sinograms may be missing views, where the missing views are grouped into sets of consecutive missing views interleaved with sets of obtained views. Each set of missing views may include at least two consecutive missing views. The multi-scale interpolation model may be a dual-channel output model, such as dual-channel output model 508, trained to output a filled HE sinogram.

The multi-scale interpolation model may have an encoder-decoder architecture with the encoder arm configured to progressively process (e.g., downsample) extracted features from the HE sinogram to a lower resolution where each set of missing or initialized views includes only one missing/initialized view (if the features were mapped back to sinogram values), similar to the encoder arm of the single-channel output model explained above with respect to FIGS. 8, 10, and 11. In the dual-channel output model, the encoder arm is also configured to progressively process (e.g., downsample) extracted features from the LE sinogram to a lower resolution where each set of missing or initialized views includes only one missing/initialized view.

Thus, at 1204, the encoder arm of the multi-scale interpolation model progressively downsamples extracted features of the HE sinogram and progressively downsamples extracted features of the LE sinogram to create N downsampled HE feature sets and N downsampled LE feature sets. The number N of downsampled feature sets that are created may depend on how many views are missing in each set of missing views of the HE and LE sinograms. If two views are missing in each set, one downsampled HE feature and one downsampled LE feature set are created. If four views are missing in each set, two downsampled HE feature sets are created and two downsampled LE feature sets are created, and so forth. The downsampling may be in the view direction, such that at each round of downsampling, two views are downsampled to one view. The downsampled feature sets that include only one missing/initialized view per original set of missing views (alternating with one downsampled obtained view) may be the final feature sets.

The final feature sets may be passed through a bottleneck layer or a super resolution layer, as explained above with respect to FIGS. 10 and 11. A first decoder arm of the multi-scale interpolation model progressively processed (e.g., upsamples) the downsampled HE feature sets, as indicated at 1208, with the model trained with a respective loss function for each resolution (as indicated at 1210) until an original resolution (e.g., matching the resolution of the original HE sinogram) HE feature set is formed, which is passed through an output layer to form the filled HE sinogram that is output from the model, as indicated at 1216. Similarly, at 1212, a second decoder arm of the multi-scale interpolation model progressively processes (e.g., upsamples) the downsampled LE feature sets, with the model trained with a respective loss function for each resolution (as indicated at 1214) until an original resolution (e.g., matching the resolution of the original LE sinogram) LE feature set is formed, which is passed through an output layer to form the filled LE sinogram that is output from the model, as indicated at 1216.

FIG. 13 schematically shows a third example architecture 1300 for a dual-channel output, multi-scale interpolation model. The multi-scale interpolation model according to the third architecture 1300 includes an encoder arm 1302 and two decoder arms: a first decoder arm 1312 and a second decoder arm 1320. The encoder arm 1302 takes as input two sinograms (e.g., an HE sinogram and an LE sinogram) each having missing views. In some examples, the input sinograms may be an initialized sinograms (e.g., initialized HE and LE sinograms). For each input sinogram, the encoder arm 1302 extracts a set of features from the input sinogram and downsamples the extracted features, as explained above with respect to the encoder arm of the architecture shown in FIG. 10. For example, at the encoder arm 132, a first downsampled feature set 1304 is formed, which is downsampled by a factor of two in the view direction to form an intermediate downsampled feature set 1306. The encoder arm 1302 downsamples the intermediate downsampled feature set 1306 by a factor of two in the view direction to form a final downsampled feature set 1308. This process is performed by the encoder arm 1302 for both the HE sinogram and the LE sinogram.

Each final downsampled feature set is passed through a bottleneck layer 1310. The output from the bottleneck layer 1310 corresponding to the HE sinogram (e.g., the most downsampled HE feature set/lowest resolution feature set of the HE sinogram) is a downsampled feature set with no missing views (e.g., where each missing/initialized view has been filled) and is passed to the first decoder arm 1312 along with the final downsampled HE feature set, where the output from the bottleneck layer is concatenated with the final downsampled HE feature set to form a first expanded HE feature set 1314. The first decoder arm upsamples the first expanded HE feature set 1314 by a factor of two and concatenates the upsampled/expanded feature set with the intermediate downsampled HE feature set to form an intermediate expanded HE feature set 1316. The first decoder arm upsamples the intermediate expanded HE feature set 1316 by a factor of two and concatenates the expanded feature set with the first downsampled HE feature set to form a final expanded HE feature set 1318. The final expanded HE feature set 1318 is passed through an output layer (e.g., 1x1 convolution) to generate the filled HE sinogram ($\hat{X}$) that is output by the model.

A similar process is performed via the second decoder arm 1320 to form the filled LE sinogram. The output from the bottleneck layer 1310 corresponding to the LE sinogram (e.g., the most downsampled LE feature set) is a downsampled feature set with no missing views (e.g., where each missing/initialized view has been filled) and is passed to the second decoder arm 1320 along with the final downsampled LE feature set, where the output from the bottleneck layer is concatenated with the final downsampled LE feature set to form a first expanded LE feature set 1322. The second decoder arm upsamples the first expanded LE feature set 1322 by a factor of two and concatenates the expanded feature set with the intermediate downsampled LE feature set to form an intermediate expanded LE feature set 1324. The second decoder arm upsamples the intermediate expanded LE feature set 1324 by a factor of two and concatenates the expanded feature set with the first downsampled LE feature set to form a final upsampled LE feature set 1326. The final upsampled LE feature set 1326 is passed through an output layer (e.g., 1x1 convolution) to generate the filled LE sinogram ($\hat{Y}$) that is output by the model.

The feature extraction, progressive processing/downsampling to decrease feature set resolution, and progressive processing/upsampling to increase feature set resolution of the multi-scale interpolation model may be performed according to weights and biases of the multi-scale interpolation model that are set during training of the multi-scale interpolation model using multiple loss functions, as explained above with respect to FIG. 10.

In addition to the loss functions calculated from each sinogram output by the first decoder arm 1312 (the output filled HE sinogram $\hat{X}$, the output intermediate interpolated HE sinogram $\hat{X}_{\downarrow 2}$, and the output first interpolated HE sinogram $\hat{X}_{\downarrow 4}$), which may be used along with the corresponding ground truth sinogram to calculate a respective loss function to update the model during training based on each loss function, further loss functions are calculated for the second decoder arm 1320. For example, the second decoder arm 1320 may output the filled LE sinogram $\hat{Y}$, the intermediate interpolated LE sinogram $\hat{Y}_{\downarrow 2}$, and the first interpolated LE sinogram $\hat{Y}_{\downarrow 4}$.

The overall loss for a round of training of the second decoder arm 1320 with N levels of downsampling may be calculated according to the following equation:

$$\mathcal{L} = \sum_{i=1}^{N} \lambda_i \mathcal{L}_i$$

The loss functions ($\mathcal{L}$) may be calculated according to the following equations:

$$\mathcal{L}_1 = \left\| \left( \hat{Y} - Y \right) \odot mask \right\|$$

$$\mathcal{L}_2 = \left\| \left( \hat{Y}_{\downarrow 2} - Y_{\downarrow 2} \right) \odot mask_{\downarrow 2} \right\|$$

$$\mathcal{L}_4 = \left\| \left( Y_{\downarrow 4} - Y_{\downarrow 4} \right) \odot mask_{\downarrow 4} \right\|$$

The losses that are calculated may be mean absolute error (MAE)/SSIM/spectral loss or feature loss.

Thus, a loss function is calculated for each sinogram/resolution level output by each decoder arm of the model based on a difference between the output sinogram and a respective ground truth sinogram. The downsampled ground truth sinograms for calculating the loss may be generated by downsampling each ground truth sinogram ($X, Y$). Further, a respective mask may be applied during each loss calculation so that the loss is calculated only on the missing/initialized views and the loss is not calculated on the obtained views, which may train the model to preserve the data in each obtained view.

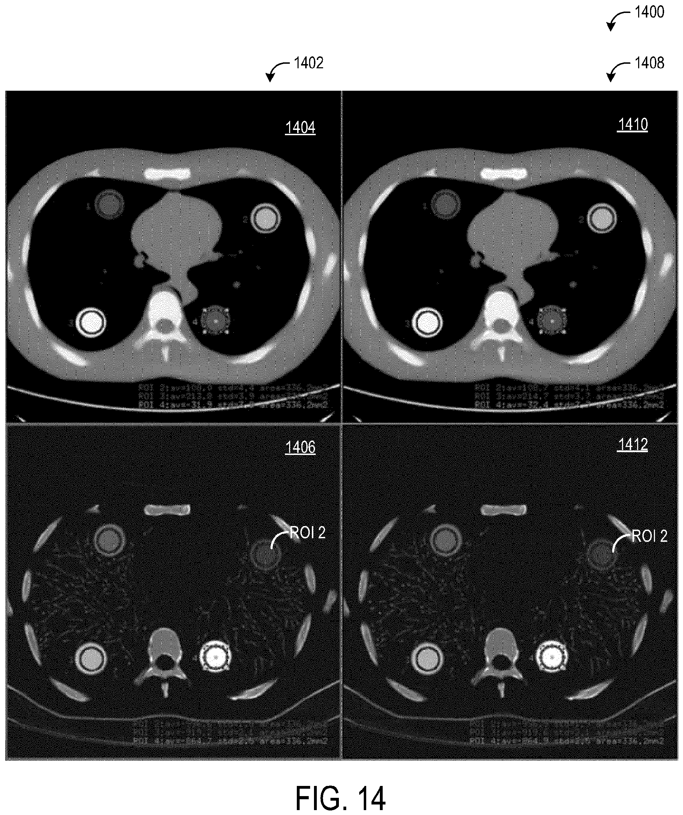

FIG. 14 shows a plurality of images 1400 that may be generated according to embodiments of the disclosure. The plurality of images 1400 includes a first set of images 1402, which may be original images reconstructed from full sinograms (e.g., no missing views in either the HE sinogram or LE sinogram). The first set of images 1402 includes material basis images, with a first image 1404 decomposed to a first basis material and a second image 1406 decomposed to a second basis material. Four regions of interest (ROIs) are marked in each image.

The plurality of images 1400 further includes a second set of images 1408, which may include images reconstructed from filled HE and LE sinograms that were originally missing views and filled using the methods disclosed herein to initialize each sinogram with data from the other sinogram and interpolate the missing views via the multi-scale interpolation model described herein. The second set of images 1408 includes material basis images, with a third image 1410 decomposed to the first basis material and a fourth image 1412 decomposed to the second basis material. The four ROIs are marked in each image.

As appreciated from FIG. 14, the second set of images 1208 are similar in quality to the first set of images 1402. As a specific example, looking at ROI 2 in the images of the second basis material (e.g., the second image 1406 and the fourth image 1412), the ROI 2 has an average value of 996.6 and a standard deviation of 1.8 in the second image 1406 (e.g., the original image of the second basis material). In comparison, for the second set of images 1408, the ROI 2 for the fourth image 1412 has an average value of 996.9 and a standard deviation of 1.9. Thus, the multi-scale interpolation models described herein can fill the missing views of sinograms that may be used to reconstruct images that are of equivalent quality to images reconstructed from sinograms without any missing views.

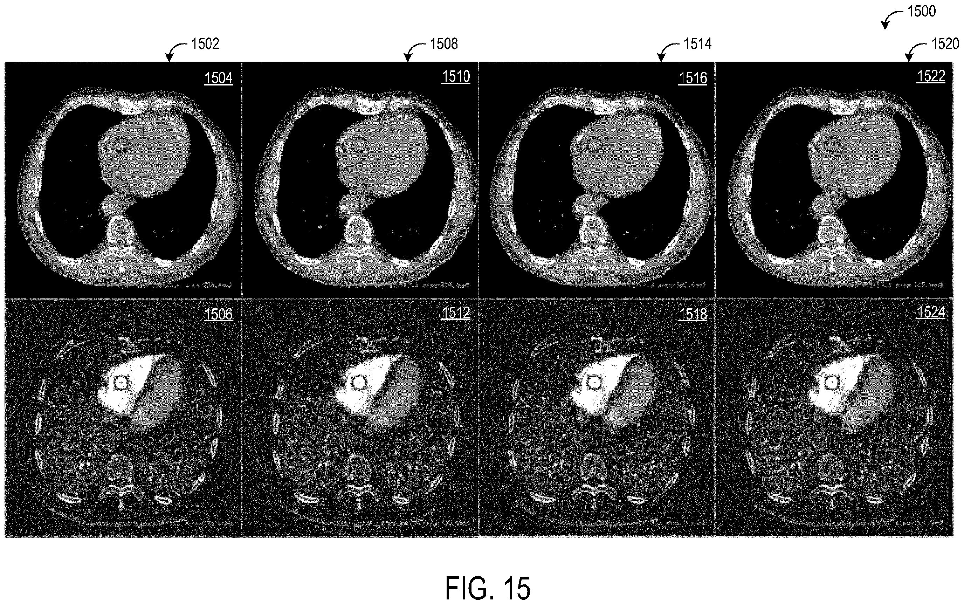

FIG. 15 shows a plurality of images 1500 that may be generated according to embodiments of the disclosure. The plurality of images 1500 includes a first set of images 1502, which may be original images reconstructed from full sinograms (e.g., no missing views in either the HE sinogram or LE sinogram). The first set of images 1502 includes material basis images, with a first image 1504 decomposed to a first basis material and a second image 1506 decomposed to a second basis material. One ROI is marked in each image.

The plurality of images 1500 further includes a second set of images 1508, a third set of images 1514, and a fourth set of images 1520, each of which may include images reconstructed from filled HE and LE sinograms that were originally missing views and filled using the methods disclosed herein to interpolate the missing views via the multi-scale interpolation model(s) described herein. However, the HE and LE sinograms used to reconstruct the second, third, and fourth sets of images may have originally included different numbers of consecutive missing views. For example, the second set of images 1508 may be reconstructed from filled HE and LE sinograms obtained with original HE and LE sinograms having groups of missing views that include four consecutive views. The third set of images 1514 may be reconstructed from filled HE and LE sinograms obtained with original HE and LE sinograms having groups of missing views that include two consecutive views. The fourth set of images 1520 may be reconstructed from filled HE and LE sinograms obtained with original HE and LE sinograms having groups of missing views that include eight consecutive views.

The second set of images 1508 includes material basis images, with a third image 1510 decomposed to the first basis material and a fourth image 1512 decomposed to the second basis material. The third set of images 1514 includes material basis images, with a fifth image 1516 decomposed to the first basis material and a sixth image 1518 decomposed to the second basis material. The fourth set of images 1520 includes material basis images, with a seventh image 1522 decomposed to the first basis material and an eighth image 1524 decomposed to the second basis material. The same ROI is marked in each image. It is to be appreciated that the same original HE and LE sinograms may be used to obtain all the images of the plurality of images 1500, such that the full HE and LE sinograms are used to reconstruct the first set of images 1502, and alternating groups of four consecutive views, two consecutive views, and eight consecutive views are removed from the full HE and LE sinograms to create the HE and LE sinograms with groups of four, two, and eight missing views that are then interpolated/filled and used to reconstruct the second, third, and fourth sets of images, respectively.

As appreciated from FIG. 15, the images reconstructed from the filled sinograms are similar in quality to the first set of images 1502 (reconstructed from the full sinograms with no missing views), regardless of how many consecutive views are missing from the pre-filled/missing view sinograms (and thus how many consecutive views have to be filled in by the multi-scale interpolation model). As a specific example, parameters of the ROI in each of the third image 1510, the fifth image 1516, and the seventh image 1522 may be similar to parameters of the ROI in the first image 1504. The ROI in the first image 1504 may have an average intensity value of 2.0 and a standard deviation of 20.4; the ROI may have an average intensity value of 1.9, 0.4, and 0.5 and a standard deviation of 17.1, 17.3, and 17.8, in the third image 1510, the fifth image 1516, and the seventh image 1522, respectively. Likewise, parameters of the ROI in each of the fourth image 1512, the sixth image 1518, and the eighth image 1524 may be similar to parameters of the ROI in the second image 1506. The ROI in the second image 1506 may have an average intensity value of 834.9 and a standard deviation of 31.1; the ROI may have an average intensity value of 835.6, 834.5, and 834.8 and a standard deviation of 30.8, 32.0, and 31.2, in the fourth image 1512, the sixth image 1518, and the eighth image 1524, respectively.

A technical effect of filling high energy and low energy sinograms, each having missing views, via a multi-scale interpolation model as described herein is that images may be reconstructed from the filled sinograms without image artifacts or image quality degradation associated with conventional interpolation techniques. Another technical effect is that any downstream processing of the filled sinograms prior to reconstruction may be reduced or avoided due to the accuracy of the multi-scale interpolation models and in particular the multiple loss functions used during training of the multi-scale interpolation models.

The disclosure also provides support for a method, comprising: obtaining a first sinogram and a second sinogram of an imaging subject, wherein the first sinogram is missing a plurality of views and the second sinogram is missing a different plurality of views, the first sinogram acquired with a first X-ray source energy during a scan and the second sinogram acquired with a second, different X-ray source energy during the scan, initializing the first sinogram with information from the second sinogram to form a first initialized sinogram, initializing the second sinogram with information from the first sinogram to form a second initialized sinogram, entering the first initialized sinogram into an interpolation model trained to output a first filled sinogram based on the first initialized sinogram, and entering the second initialized sinogram into the interpolation model or another interpolation model trained to output a second filled sinogram based on the second initialized sinogram, and reconstructing one or more images from the first filled sinogram and the second filled sinogram. In a first example of the method, entering the first initialized sinogram into the interpolation model and entering the second initialized sinogram into the interpolation model or another interpolation model comprises entering both the first initialized sinogram and the second initialized sinogram into the interpolation model, the interpolation model comprising a dual-channel output model trained to output both the first filled sinogram and the second filled sinogram. In a second example of the method, optionally including the first example, entering the first initialized sinogram into the interpolation model and entering the second initialized sinogram into the interpolation model or another interpolation model comprises entering the first initialized sinogram into the interpolation model and entering the second initialized sinogram into the other interpolation model, the interpolation model comprising a first single-channel output model trained to output the first filled sinogram and the other interpolation model comprising a second single-channel output model trained to output the second filled sinogram. In a third example of the method, optionally including one or both of the first and second examples, the plurality of views missing from the first sinogram includes multiple sets of consecutive missing views alternating with multiple sets of obtained views, and wherein initializing the first sinogram comprises, for each set of consecutive missing views of the multiple sets of consecutive missing views, identifying complementary views from the second sinogram, scaling sinogram data of each view of the complementary views from the second sinogram, and filling in each set of consecutive missing views with respective scaled sinogram data to form the first initialized sinogram. In a fourth example of the method, optionally including one or more or each of the first through third examples, initializing the first sinogram comprises: processing the first sinogram through a first set of operations and downsampling to generate a first set of features, the first set of features comprising higher-dimensional features corresponding the first sinogram, processing the second sinogram through a second set of operations and downsampling to generate a second set of features, the second set of features comprising higher-dimensional features corresponding the second sinogram, mixing the first set of features and the second set of features to form a mixed set of features, and upsampling the mixed set of features to form the first initialized sinogram. In a fifth example of the method, optionally including one or more or each of the first through fourth examples, the interpolation model includes an encoder arm configured to progressively process features extracted from the first initialized sinogram until a final processed feature set is formed. In a sixth example of the method, optionally including one or more or each of the first through fifth examples, the interpolation model further includes a bottleneck layer or a super resolution layer. In a seventh example of the method, optionally including one or more or each of the first through sixth examples, the interpolation model further includes a decoder arm configured to progressively process the final processed feature set until a final expanded feature set is formed, which is passed through an output layer to form the first filled sinogram. In an eighth example of the method, optionally including one or more or each of the first through seventh examples, the encoder arm is configured to pass each processed feature set to the decoder arm and the decoder arm is configured to progressively process the final processed feature set using each processed feature set. In a ninth example of the method, optionally including one or more or each of the first through eighth examples, the interpolation model is trained with a plurality of loss functions, each loss function calculated from output from the decoder arm at a respective stage of the progressive processing of the decoder arm.

The disclosure also provides support for an imaging system, comprising: an X-ray source that emits a beam of X-rays toward a subject to be imaged, a detector that receives the beam of X-rays attenuated by the subject, a data acquisition system (DAS) operably connected to the detector , and memory storing instructions and one or more processors configured to execute the instructions to: obtain a first sinogram and a second sinogram of the subject from the DAS, wherein the first sinogram is missing a plurality of views and the second sinogram is missing a different plurality of views, the first sinogram acquired with the X-ray source operated at a first X-ray source energy during a scan and the second sinogram acquired with the X-ray source operated at a second, different X-ray source energy during the scan, initialize the first sinogram with information from the second sinogram to form a first initialized sinogram, initialize the second sinogram with information from the first sinogram to form a second initialized sinogram, enter the first initialized sinogram into an interpolation model trained to output a first filled sinogram based on the first initialized sinogram enter the second initialized sinogram into the interpolation model or another interpolation model trained to output a second filled sinogram based on the second initialized sinogram, and reconstruct one or more images from the first filled sinogram and the second filled sinogram. In a first example of the system, the plurality of views missing from the first sinogram includes multiple sets of consecutive missing views alternating with multiple sets of obtained views, and wherein initializing the first sinogram comprises adding information the second sinogram to each missing view of the first sinogram such that the first initialized sinogram includes multiple sets of consecutive initialized views alternating with the multiple sets of obtained views. In a second example of the system, optionally including the first example, the interpolation model is configured to: progressively process features extracted from the first initialized sinogram via multiple processing stages to form a final processed feature set where each set of consecutive initialized views includes only one initialized view, and progressively process the final processed feature set via multiple further processing stages until a final expanded feature set is formed and passed through an output layer to form the first filled sinogram. In a third example of the system, optionally including one or both of the first and second examples, the interpolation model is trained with a plurality of loss functions, each loss function calculated at a respective further processing stage.

The disclosure also provides support for a method, comprising: obtaining a first sinogram and a second sinogram of an imaging subject, wherein the first sinogram is missing a plurality of views and the second sinogram is missing a different plurality of views, the first sinogram acquired with a first X-ray source energy during a scan and the second sinogram acquired with a second, different X-ray source energy during the scan, obtaining a first filled sinogram output from an interpolation model trained to output the first filled sinogram based on the first sinogram, the interpolation model configured to perform multiple stages of interpolation in order to fill in each of the plurality of views missing from the first sinogram, where the interpolation model is trained with a respective loss function at each stage, obtaining a second filled sinogram output from the interpolation model or another interpolation model trained to output the second filled sinogram based on the second sinogram, and reconstructing one or more images from the first filled sinogram and the second filled sinogram. In a first example of the method, the plurality of views missing from the first sinogram includes multiple sets of consecutive missing views alternating with multiple sets of obtained views, and wherein the interpolation model includes a super resolution layer configured to receive features extracted from the first sinogram at a lowest resolution where each set of consecutive missing views includes only missing view and output an upsampled feature set at a higher resolution where each missing view is filled with data. In a second example of the method, optionally including the first example, obtaining the second filled sinogram comprises obtaining the second filled sinogram output from the interpolation model, the interpolation model comprising a dual-channel output model trained to output both the first filled sinogram and the second filled sinogram. In a third example of the method, optionally including one or both of the first and second examples, the interpolation model includes an encoder arm, a first decoder arm, and a second decoder arm, the first decoder arm configured to output the first filled sinogram and the second decoder arm configured to output the second filled sinogram. In a fourth example of the method, optionally including one or more or each of the first through third examples, the encoder arm is configured to: receive, as input, an initialized version of the first sinogram and an initialized version of the second sinogram, progressively process, via multiple processing stages, a first set of features extracted from the initialized version of the first sinogram and pass each processed first set of features of the first sinogram to the first decoder arm, and progressively process, via the multiple processing stages, a second set of features extracted from the initialized version of the second sinogram and pass each processed second set of features of the second sinogram to the second decoder arm. In a fifth example of the method, optionally including one or more or each of the first through fourth examples, the method further comprises: generating the initialized version of the first sinogram by performing feature-level mixing of the first sinogram and the second sinogram or adding scaled sinogram data from the second sinogram to the first sinogram, and generating the initialized version of the second sinogram by performing feature-level mixing of the second sinogram and the first sinogram or adding scaled sinogram data from the first sinogram to the second sinogram.

As used herein, an element or step recited in the singular and proceeded with the word "a" or "an" should be understood as not excluding plural of said elements or steps, unless such exclusion is explicitly stated. Furthermore, references to "one embodiment" of the present invention are not intended to be interpreted as excluding the existence of additional embodiments that also incorporate the recited features. Moreover, unless explicitly stated to the contrary, embodiments "comprising," "including," or "having" an element or a plurality of elements having a particular property may include additional such elements not having that property. The terms "including" and "in which" are used as the plain-language equivalents of the respective terms "comprising" and "wherein." Moreover, the terms "first," "second," and "third," etc. are used merely as labels, and are not intended to impose numerical requirements or a particular positional order on their objects.

This written description uses examples to disclose the invention, including the best mode, and also to enable a person of ordinary skill in the relevant art to practice the invention, including making and using any devices or systems and performing any incorporated methods. The patentable scope of the invention is defined by the claims, and may include other examples that occur to those of ordinary skill in the art. Such other examples are intended to be within the scope of the claims if they have structural elements that do not differ from the literal language of the claims, or if they include equivalent structural elements with insubstantial differences from the literal languages of the claims.

**Claims**

1. A method, comprising:

    obtaining (302, 304) a first sinogram and a second sinogram of an imaging subject, wherein the first sinogram is missing a plurality of views and the second sinogram is missing a different plurality of views, the first sinogram acquired with a first X-ray source energy during a scan and the second sinogram acquired with a second, different X-ray source energy during the scan;
    initializing (306) the first sinogram with information from the second sinogram to form a first initialized sinogram;

initializing (306) the second sinogram with information from the first sinogram to form a second initialized sinogram;

entering (312) the first initialized sinogram into an interpolation model trained to output a first filled sinogram based on the first initialized sinogram, and entering (312) the second initialized sinogram into the interpolation model or another interpolation model trained to output a second filled sinogram based on the second initialized sinogram; and

reconstructing (318) one or more images from the first filled sinogram and the second filled sinogram.

2. The method of claim 1, wherein entering the first initialized sinogram into the interpolation model and entering the second initialized sinogram into the interpolation model or another interpolation model comprises entering (314) both the first initialized sinogram and the second initialized sinogram into the interpolation model, the interpolation model comprising a dualchannel output model trained to output both the first filled sinogram and the second filled sinogram.

3. The method of claim 1, wherein entering the first initialized sinogram into the interpolation model and entering the second initialized sinogram into the interpolation model or another interpolation model comprises entering (316) the first initialized sinogram into the interpolation model and entering the second initialized sinogram into the other interpolation model, the interpolation model comprising a first single-channel output model trained to output the first filled sinogram and the other interpolation model comprising a second single-channel output model trained to output the second filled sinogram.

4. The method of claim 1, wherein the plurality of views missing from the first sinogram includes multiple sets of consecutive missing views alternating with multiple sets of obtained views, and wherein initializing (310) the first sinogram comprises, for each set of consecutive missing views of the multiple sets of consecutive missing views, identifying complementary views from the second sinogram, scaling sinogram data of each view of the complementary views from the second sinogram, and filling in each set of consecutive missing views with respective scaled sinogram data to form the first initialized sinogram.

5. The method of claim 1, wherein initializing the first sinogram comprises:

    processing (608) the first sinogram through a first set of operations and downsampling to generate a first set of features, the first set of features comprising higher-dimensional features corresponding the first sinogram;

processing (608) the second sinogram through a second set of operations and downsampling to generate a second set of features, the second set of features comprising higher-dimensional features corresponding the second sinogram;

mixing (610) the first set of features and the second set of features to form a mixed set of features; and

upsampling (612) the mixed set of features to form the first initialized sinogram.

6. The method of claim 1, wherein the interpolation model includes an encoder arm (1002) configured to progressively process features extracted from the first initialized sinogram until a final processed feature set is formed.

7. The method of claim 6, wherein the interpolation model further includes a bottleneck layer (1010) or a super resolution layer (1110).

8. The method of claim 6, wherein the interpolation model further includes a decoder arm (1012) configured to progressively process the final processed feature set until a final expanded feature set is formed, which is passed through an output layer to form the first filled sinogram.

9. The method of claim 8, wherein the encoder arm is configured to pass each processed feature set to the decoder arm and the decoder arm is configured to progressively process the final processed feature set using each processed feature set.

10. The method of claim 9, wherein the interpolation model is trained with a plurality of loss functions, each loss function calculated from output from the decoder arm at a respective stage of the progressive processing of the decoder arm.

11. An imaging system, comprising:

    an X-ray source (104) that emits a beam of X-rays toward a subject to be imaged;
    a detector (108) that receives the beam of X-rays attenuated by the subject;
    a data acquisition system (DAS) (214) operably connected to the detector; and
    memory storing instructions and one or more processors (216) configured to execute the instructions to:

        obtain (302, 304) a first sinogram and a second sinogram of the subject from the DAS, wherein the first sinogram is missing a plurality of views and the second sinogram is missing a different plurality of views, the first sinogram acquired with the X-ray source operated at a first X-ray source energy during a scan and the second sinogram acquired with the X-ray source operated at a second, different X-ray source energy during the scan;

initialize (306) the first sinogram with information from the second sinogram to form a first initialized sinogram;

initialize (306) the second sinogram with information from the first sinogram to form a second initialized sinogram;

enter (312) the first initialized sinogram into an interpolation model trained to output a first filled sinogram based on the first initialized sinogram;

enter (312) the second initialized sinogram into the interpolation model or another interpolation model trained to output a second filled sinogram based on the second initialized sinogram; and

reconstruct (318) one or more images from the first filled sinogram and the second filled sinogram.

12. The system of claim 11, wherein the plurality of views missing from the first sinogram includes multiple sets of consecutive missing views alternating with multiple sets of obtained views, and wherein initializing the first sinogram comprises adding information the second sinogram to each missing view of the first sinogram such that the first initialized sinogram includes multiple sets of consecutive initialized views alternating with the multiple sets of obtained views.

13. The system of claim 12, wherein the interpolation model is configured to:

progressively process (804) features extracted from the first initialized sinogram via multiple processing stages to form a final processed feature set where each set of consecutive initialized views includes only one initialized view; and

progressively process (808) the final processed feature set via multiple further processing stages until a final expanded feature set is formed and passed through an output layer to form the first filled sinogram.

14. The system of claim 13, wherein the interpolation model is trained with a plurality of loss functions, each loss function calculated at a respective further processing stage.

FIG. 1

FIG. 2

300

START

↓

302

OBTAIN HIGH ENERGY (HE) SINOGRAM

↓

304

OBTAIN LOW ENERGY (LE) SINOGRAM

↓

306

INITIALIZE HE SINOGRAM WITH DATA FROM LE SINOGRAM AND INITIALIZE LE SINOGRAM WITH DATA FROM HE SINOGRAM

FEATURE-LEVEL MIXING 308

INPUT-LEVEL MIXING 310

↓

312

OBTAIN FILLED HE SINOGRAM AND FILLED LE SINOGRAM BASED ON (INITIALIZED) SINOGRAMS

VIA DUAL CHANNEL OUTPUT MODEL 314

VIA TWO SINGLE CHANNEL OUTPUT MODELS 316

↓

318

RECONSTRUCT ONE OR MORE IMAGES FROM FILLED HE SINOGRAM AND FILLED LE SINOGRAM

↓

END

FIG. 3

FIG. 4

HE SINOGRAM 502

LE SINOGRAM 504

506

DUAL-CHANNEL MODEL 508

FILLED HE SINOGRAM 510

FILLED LE SINOGRAM 512

500

FIG. 5

600

START

602

PRE-PROCESS HE SINOGRAM

604

PRE-PROCESS LE SINOGRAM

606

PERFORM FIRST FEATURE LEVEL MIXING
TO GENERATE INITIALIZED HE SINOGRAM

PROCESS HE AND LE SINOGRAMS
SEPARATELY TO OBTAIN TWO
SETS OF FEATURES 608

MIX FEATURES AT MIXING LAYER
610

PERFORM CONVOLUTIONS AND
UPSAMPLING 612

614

PERFORM SECOND FEATURE LEVEL
MIXING TO GENERATE INITIALIZED LE
SINOGRAM

END

FIG. 6

FIG. 7

EP 4 413 928 A1

800

START

802

ENTER (INITIALIZED) HE SINOGRAM AS
INPUT TO MODEL

804

ENCODER ARM PROGRESSIVELY
DOWNSAMPLES FEATURES FROM
(INITIALIZED) HE SINOGRAM TO CREATE
N DOWNSAMPLED FEATURE SETS

806

PASS FINAL FEATURE SET TO DECODER
ARM VIA LOWEST LAYER

808

DECODER ARM PROGRESSIVELY
UPSAMPLES FEATURE SETS

MODEL TRAINED WITH LOSS
FUNCTION FOR RESOLUTION 810

812

RECEIVE FILLED HE SINOGRAM OUTPUT
FROM MODEL

END

FIG. 8

FIG. 9

FIG. 10

FIG. 11

1200

```
        ┌──────────┐
        │  START   │
        └──────────┘
             │
             ▼                              ╭─ 1202
┌─────────────────────────────────┐
│ ENTER (INITIALIZED) HE SINOGRAM AND│
│ (INITIALIZED) LE SINOGRAM AS INPUT TO│
│              MODEL               │
└─────────────────────────────────┘
             │
             ▼                              ╭─ 1204
┌─────────────────────────────────┐
│     ENCODER ARM PROGRESSIVELY    │
│      DOWNSAMPLES FEATURES FROM   │
│  (INITIALIZED) HE SINOGRAM AND LE│
│   SINOGRAM TO CREATE N           │
│  DOWNSAMPLED HE AND LE FEATURE   │
│              SETS                │
└─────────────────────────────────┘
             │
             ▼                              ╭─ 1206
┌─────────────────────────────────┐
│ FIRST DECODER ARM PROGRESSIVELY  │
│     UPSAMPLES DOWNSAMPLED HE     │
│          FEATURE SETS            │
│ ┌ ─ ─ ─ ─ ─ ─ ─ ─ ─ ─ ─ ─ ─ ─ ┐ │
│ │   MODEL TRAINED WITH LOSS   │ │
│ │ FUNCTION FOR EACH RESOLUTION│ │
│ │             1208            │ │
│ └ ─ ─ ─ ─ ─ ─ ─ ─ ─ ─ ─ ─ ─ ─ ┘ │
└─────────────────────────────────┘
             │
             ▼                              ╭─ 1210
┌─────────────────────────────────┐
│       SECOND DECODER ARM         │
│     PROGRESSIVELY UPSAMPLES      │
│    DOWNSAMPLED LE FEATURE SETS   │
│ ┌ ─ ─ ─ ─ ─ ─ ─ ─ ─ ─ ─ ─ ─ ─ ┐ │
│ │   MODEL TRAINED WITH LOSS   │ │
│ │ FUNCTION FOR EACH RESOLUTION│ │
│ │             1212            │ │
│ └ ─ ─ ─ ─ ─ ─ ─ ─ ─ ─ ─ ─ ─ ─ ┘ │
└─────────────────────────────────┘
             │
             ▼                              ╭─ 1214
┌─────────────────────────────────┐
│  RECEIVE FILLED HE SINOGRAM AND  │
│ FILLED LE SINOGRAM OUTPUT FROM   │
│              MODEL               │
└─────────────────────────────────┘
             │
             ▼
        ┌──────────┐
        │   END    │
        └──────────┘
```

FIG. 12

FIG. 13

FIG. 14

FIG. 15

EP 4 413 928 A1

Europäisches
Patentamt

European
Patent Office

Office européen
des brevets

# EUROPEAN SEARCH REPORT

Application Number

EP 24 15 1592

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2023/003924 A1 (LEO CANCER CARE INC [US]) 26 January 2023 (2023-01-26) * page 28 - page 31; figures 20, 22 * | 1-14 | INV. A61B6/00 G06T11/00 A61B6/03 G06T5/50 G06T5/60 |
| A | US 2021/290193 A1 (ZHOU JIAN [US] ET AL) 23 September 2021 (2021-09-23) * paragraph [0041] - paragraph [0128]; figures 1-3, 7 * | 1-14 | |
| A | US 2022/292743 A1 (FIRSCHING MARKUS [DE] ET AL) 15 September 2022 (2022-09-15) * paragraph [0095] - paragraph [0164]; figures 1, 2 * | 1-14 | |
| A | LEE HOYEON ET AL: "View-interpolation of sparsely sampled sinogram using convolutional neural network", PROGRESS IN BIOMEDICAL OPTICS AND IMAGING, SPIE - INTERNATIONAL SOCIETY FOR OPTICAL ENGINEERING, BELLINGHAM, WA, US, vol. 10133, 24 February 2017 (2017-02-24), pages 1013328-1013328, XP060086962, ISSN: 1605-7422, DOI: 10.1117/12.2254244 ISBN: 978-1-5106-0027-0 * abstract * * page 2 - page 4; figure 1 * | 1-14 | |

TECHNICAL FIELDS
SEARCHED (IPC)

A61B
G06T

The present search report has been drawn up for all claims

| Place of search | Date of completion of the search | Examiner |
|---|---|---|
| The Hague | 1 July 2024 | Lauritzen, Jan |

EPO FORM 1503 03.82 (P04C01)

## ANNEX TO THE EUROPEAN SEARCH REPORT
## ON EUROPEAN PATENT APPLICATION NO.

EP 24 15 1592

This annex lists the patent family members relating to the patent documents cited in the above-mentioned European search report.
The members are as contained in the European Patent Office EDP file on
The European Patent Office is in no way liable for these particulars which are merely given for the purpose of information.

01-07-2024

| Patent document cited in search report | Publication date | Patent family member(s) | Publication date |
|---|---|---|---|
| WO 2023003924 A1 | 26-01-2023 | AU 2022313161 A1<br>EP 4373405 A1<br>US 2023038970 A1<br>WO 2023003924 A1 | 25-01-2024<br>29-05-2024<br>09-02-2023<br>26-01-2023 |
| US 2021290193 A1 | 23-09-2021 | CN 111345835 A<br>JP 7334048 B2<br>JP 2020099662 A<br>US 2020196972 A1<br>US 2021290193 A1 | 30-06-2020<br>28-08-2023<br>02-07-2020<br>25-06-2020<br>23-09-2021 |
| US 2022292743 A1 | 15-09-2022 | EP 4057229 A1<br>JP 7318045 B2<br>JP 2022140410 A<br>US 2022292743 A1 | 14-09-2022<br>31-07-2023<br>26-09-2022<br>15-09-2022 |

EPO FORM P0459

For more details about this annex : see Official Journal of the European Patent Office, No. 12/82